# EUROPEAN PATENT APPLICATION

(11) **EP 3 128 006 A1**
(43) Date of publication of application: **08.02.2017**
(21) Application number: 15179986.3
(22) Date of filing: 06.08.2015
(51) Int. Cl.: C12N 15/00

(54) **TOOLS FOR MULTIPROTEIN COMPLEX EXPRESSION IN PICHIA PASTORIS**

(71) Applicant: Consejo Superior de Investigaciones Cientificas (CSIC), 28006 Madrid (ES); EMBL (European Molecular Biology Laboratory), 69117 Heidelberg (DE)
(72) Inventor: VEGA FERNÁNDEZ, María Cristina, 28040 Madrid (ES); FERNÁNDEZ PÉREZ, Francisco José, 28040 Madrid (ES); BERGER, Imre, 38120 St. Egreve (FR)
(74) Representative: ZBM Patents - Zea, Barlocci & Markvardsen

(57) **Abstract**

The invention provides an isolated multiple integration element (MIE) comprising, in the specified order: a) a homing endonuclease site (HE); b) a promoter selected from the group consisting of *AOX1, FLD1, FMD1, GAP* and *TEF1;* c) a BstBI restriction site; d) a sequence encoding a secretion signal; e) a Ncol restriction site; f) a selection marker gene; g) a Pmel restriction site; h) a DNA sequence encoding a detection and/or purification polypeptide; i) a terminator selected from the group consisting of *AOX1, FLD1, FMD1, GAP* and *TEF1;* and j) a BstXI restriction site, wherein the HE and BstXI sites are selected such that HE and BstXI result in compatible cohesive ends when cut by the homing endonuclease and the BstXI restriction enzyme, respectively, and the ligation product of HE and BstXI cohesive ends can neither be cleaved by the homing endonuclease nor the BstXI restriction enzyme.

## Description

The present invention relates to the field of molecular biology, particularly to protein expression systems. The invention provides tools for multiprotein complex expression in *Pichia pastoris.*

### BACKGROUND ART

The recombinant production of multiprotein complexes is a time-consuming, labor-intensive and costly endeavor that is nonetheless crucial for modern biology (from biotechnology to systems biology) owing to the fact that nearly all biological functions are performed by protein complexes with several subunits.

A limited number of systems have been introduced in recent years for simultaneous expression of several genes in eukaryotic and prokaryotic hosts (see, e.g. Fitzgerald et al., Nat. Methods 2006). In spite of considerable improvements of eukaryotic expression systems, in particular the baculovirus/insect cell expression (Fitzgerald et al. (2006), *infra), E. coli* still remains to date the dominant workhorse in most laboratories, for many good reasons such as low-cost and availability of a multitude of specialized expression strains. The current co-expression systems for *E. coli* rely essentially on serial, mostly conventional (i.e. restriction/ligation) subcloning of encoding genes either as single expression or as polycistrons constituting several genes under the control of the same promoter. This considerably limits the applicability of these co-expression techniques for production of protein complexes with many subunits, in particular at the throughput typically required for structural molecular biology.

Berger *et al* have recently developed a new method for producing multiple protein complexes *(*Nat Methods 2009, WO2010100278, *infra)* in *E. coli* as well as in insect cells through baculovirus. The method makes use of donor and acceptor vectors, each containing a multiple integration element (MIE) flanked by a homing endonuclease recognition site (acceptor vectors: I-Ceul; donor vectors: PI-SceI) and a complementary BstXI site. Homing endonucleases are rare cutters with long (20-30 bp) recognition sequences that are unique even in very large DNAs. Digestion by homing endonuclease gives rise to a specific overhang that matches a corresponding BstXI site. This can be used to generate multiple expression cassettes iteratively by insertion of expression cassettes liberated by homing endonuclease and BstXI digestion into constructs linearized at the homing endonuclease site.

Donor and acceptor vectors disclosed by Berger et al (supra) additionally comprise a IoxP imperfect inverted repeat which allows for Cre recombinase-mediated fusion of said vectors. Interestingly, donor and acceptor vectors carrying multiple expression cassettes generated following the procedure explained above may be fused in the presence of Cre recombinase to rapidly create plasmids containing a large number of genes of interest. The authors demonstrate that this system provides for efficient expression of multiprotein complexes in *E. coli* and insect hosts.

However, the vectors described above are not suitable for expression in other hosts of interest. In particular, these vectors may not be used for multiprotein expression in yeasts. The use of yeasts, in particular, *Pichia pastoris,* as a cellular host for the expression of recombinant proteins has become increasingly popular. Being a eukaryote, *P. pastoris* has the advantage, when compared to *E. coli,* that it provides the potential for producing soluble, correctly folded eukaryotic recombinant proteins that have undergone post-translational modifications required for functionality. Additionally, *P. pastoris* may rapidly produce recombinant proteins at higher yields and lower costs when compared to other eukaryotic hosts and, besides, it is easier to genetically manipulate and culture than, for example, mammalian cells. *P*. *pastoris* is thus often the protein expression host of choice for the production of biopharmaceuticals. And yet, appropriate tools for expression of multiprotein complexes in *P. pastoris* are notably lacking.

In view of the above, there is a need to provide tools allowing for rapid, facile and versatile assembly of multigene constructs in *P. pastoris* for effective expression of multiprotein complexes.

### SUMMARY OF THE INVENTION

The inventors have developed new tools that enable facile and efficient expression of multiprotein complexes in *Pichia pastoris.* Said tools are in the form of nucleic acids that comprise a MIE specially designed to generate very complex multigene constructs for multiprotein expression in *P. pastoris* in a rapid and convenient manner.

In a first aspect the invention refers to an isolated multiple integration element (MIE) comprising, in the specified order: a) a homing endonuclease site (HE); b) a promoter sequence selected from the group consisting of *AOX1, FLD1, FMD1, GAP* and *TEF1,* wherein the promoter sequence is devoid of HE, BstBI, Ncol, Pmel and BstXI restriction sites if said restriction sites are present; c) a BstBI restriction site; d) a sequence encoding a secretion signal; e) an Ncol restriction site; f) a selection marker gene; g) a Pmel restriction site; h) a sequence encoding a detection and/or purification polypeptide; i) a terminator sequence selected from the group consisting of *AOX1, FLD1, FMD1, GAP* and *TEF1,* wherein the terminator sequence is devoid of HE, BstBI, Ncol, Pmel and BstXI restriction sites if said restriction sites are present; and j) a BstXI restriction site, wherein the HE and BstXI sites are selected such that HE and BstXI result in compatible cohesive ends when cut by the homing endonuclease and the BstXI restriction enzyme, respectively, and the ligation product of HE and BstXI cohesive ends can neither be cleaved by the homing endonuclease nor the BstXI restriction enzyme.

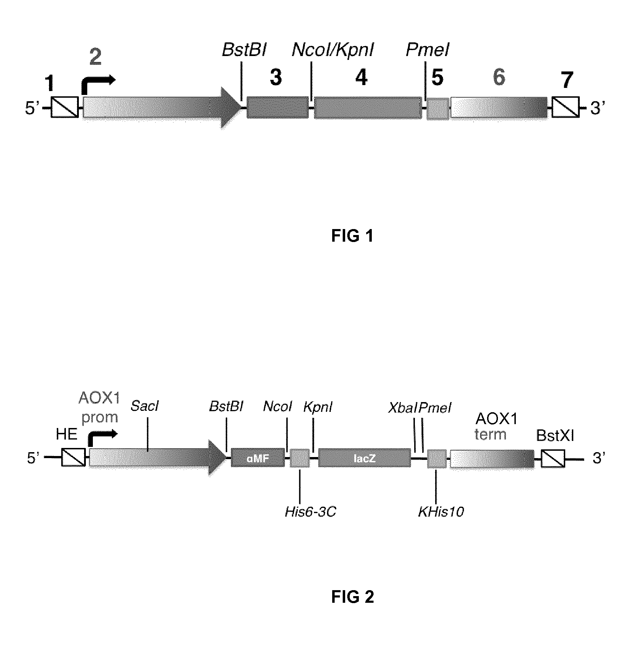

The MIE of the first aspect of the invention may be represented as disclosed in Figure 1.

This design provides several advantageous functionalities:
1- The MIE of the invention has been optimized for ligase-independent cloning strategies such as SLIC/Gibson/InFusion cloning. This provides for:
   (a) efficient and cost-effective parallel vector construction, (b) sequence-independent insertion, (c) rapid, single-step reaction, (d) precise engineering of constructs, with no additional vector or restriction site-derived amino acids added to the expressed protein, and (e) efficient insertion over a wide insert size and concentration range, all of which, together with the introduction of a selection marker, allows for high-throughput cloning of genes and rapid selection of clones containing the gene of interest for evaluating expression performance.
2- Rapid and facile addition of secretion signals and purification tags by using restriction sites especially reserved for this purpose. The versatility of the tool enabling the user to select between intracellular expression and secretion of the gene of interest is particularly advantageous. The state of the art in *P*. *pastoris* vectors is that two separate vectors are necessary, one with the alpha-MF leader sequence, which, when fused to the gene of interest leads to the secretion of the expressed protein product; and another vector, without that sequence, for intracellular expression. High-throughput cloning and efficiency is greatly enhanced by the specific design of the MIE of the invention, because one single vector can be used for both purposes. Hence, the MIE of the invention has a secretion signal sequence with a BstBI restriction site between the promoter and the secretion signal sequence, and an Ncol site right after the secretion signal sequence. Cloning a gene of interest for intracellular expression involves linearizing the vector which comprises the MIE using BstBI and a second restriction site, while cloning it for secretion expression involves digesting the vector with Ncol and a second restriction site.
3- Assembly of multigene constructs by concatamerization of genes using HE/BstXI-digested cassettes containing the gene of interest into HE/BstXI.

In order to arrive at a MIE which provides all the above advantageous functionalities, the inventors have overcome multiple practical difficulties. For example, appropriate sequence elements had to be selected, the most convenient orientation had to be devised for the various elements and adequate restriction sites needed to be selected and placed in the convenient order. Further, due care is required to maintain all sequence elements working together, which brings the difficulty that introduction/modification of one element might result in dysfunction of the system because of defective interaction with other sequence elements. This is illustrated, for instance, by the fact that the promoter and terminator need to be modified to eliminate the HE, BstBI, Ncol, Pmel and BstXI restriction sites, whenever said restriction sites are present in the promoter/terminator sequence. And all this keeping in mind the ultimate goal of the system, which is to allow for cloning of multigene constructs in *P. pastoris* while preserving the integrity and the functionality expected from the expression system.

The above MIE may be used to obtain an expression cassette by inserting one or more genes of interest (GOI) between the HE and the BstXI sites following any of the available cloning techniques. The resulting expression cassette, flanked by the HE and BstXI restriction sites, may be then used to generate multigene constructs containing multiple expression cassettes by any of the methods explained below. Thus, aspect of the invention refers to a method for obtaining an expression cassette that comprises inserting one or more GOI into a MIE as defined above and to the expression cassette directly obtained by said method. This aspect can also be formulated as an expression cassette derived from the MIE as defined above comprising one or more GOI.

The MIE or the expression cassette of the invention may be conveniently contained in a plasmid or vector. Thus, a third aspect of the invention refers to a plasmid or vector comprising the MIE according to the first aspect of the invention or the expression cassette according to the second aspect of the invention. The invention also provides a host cell containing the expression cassette or the vector according to the first and second aspects of the invention.

Another aspect of the invention relates to a kit for cloning and/or expression of multiprotein complexes containing at least one vector as defined above.

The present invention also relates to a method for producing a vector containing multiple expression cassettes comprising the steps of: (a) providing a first vector comprising an expression cassette according to the present invention; (b) providing a second vector comprising an expression cassette according to the present invention; (c) cleaving the first vector with a homing endonuclease specific for site HE and with BstXI yielding a first expression cassette flanked by the cleaved HE and BstXI sites; (d) cleaving the second vector with a homing endonuclease specific for site HE; (e) ligating the fragment obtained in step (c) into the cleaved second vector obtained in step (d) generating a third vector; and optionally, (f) repeating steps (a) to (e) with one or more vector(s) generating a vector containing multiple expression cassettes.

The present invention is further directed to a method for producing multiple protein complexes comprising the steps of: (i) producing a vector comprising from 1 to 10 expression cassettes as defined herein; (ii) introducing the vector obtained in step (i) into a *P. pastoris* host cell; and (iii) incubating the *P*. *pastoris* host cell under conditions allowing the simultaneous expression of the genes present in the vector.

### BRIEF DESCRIPTION OF THE DRAWINGS

FIG 1: Map of the MIE of the invention. HE (1), promoter (2), secretion signal (3), marker/selection cassette (4), detection and/or purification polypeptide (5), terminator (6), BstXI (7).
FIG 2: Map of a particular embodiment of the MIE of the invention. alpha-MF: S. *cerevisiae* alpha-mating factor; His 6-3C: N-terminal hexahistidine-3C tag; KHis10: C-terminal lysine-decahistidine.

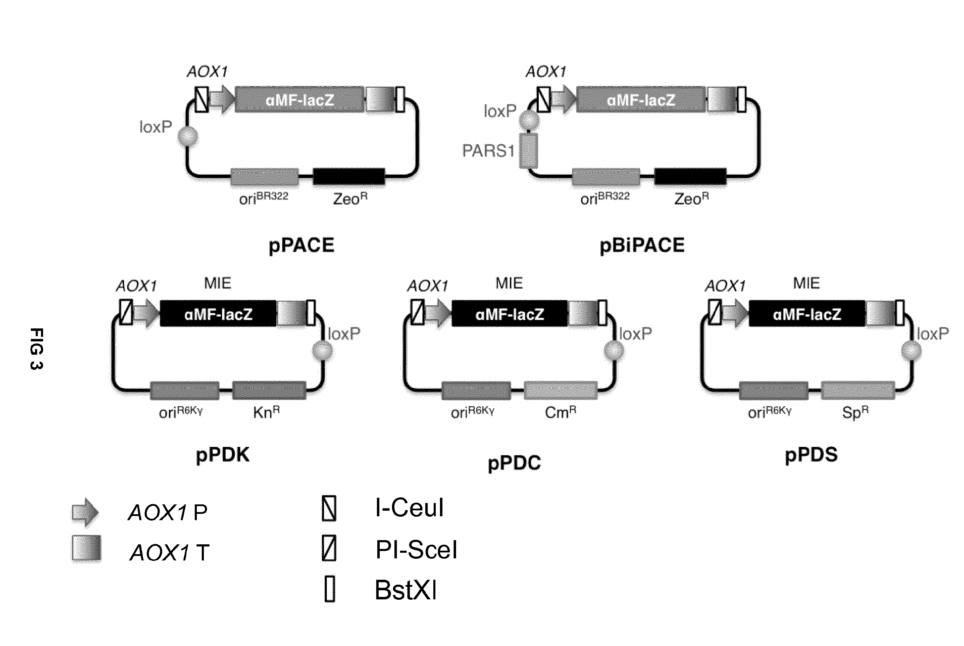

FIG 3: Schematic vector maps for pPACE (acceptor), pBiPACE (acceptor), pPDK (donor), pPDC (donor) and pPDS (donor). *AOX1* P: *AOX1* Promoter; *AOX1* T: *AOX1* Terminator.

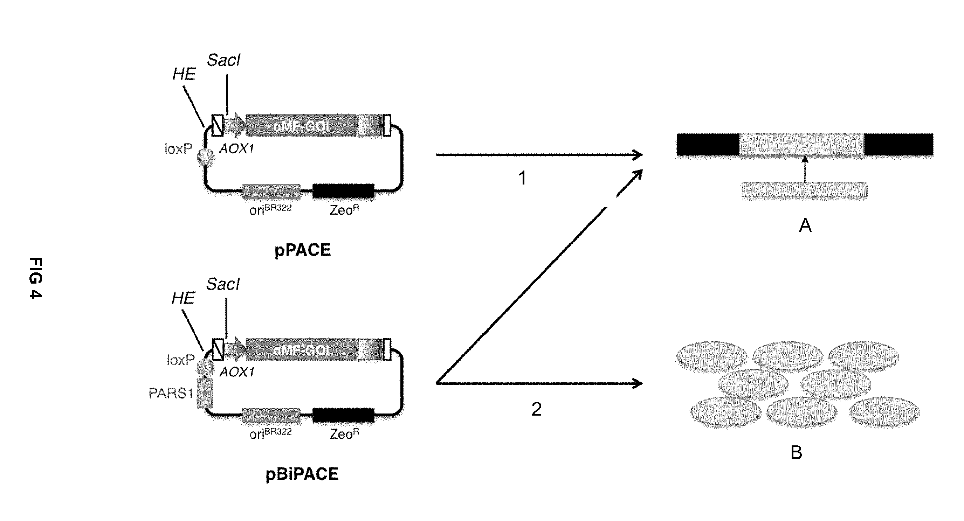

FIG 4: Transformation approaches for pPACE and pBiPACE Acceptor vectors. 1: digestion with SacI/HE and electroporation. 2: Direct transformation of circular plasmid. A: One or more copies of linearized plasmid become integrated into *P*. *pastoris* genome at AOX1 locus. B: Multiple copies of circular plasmid are maintained episomally.

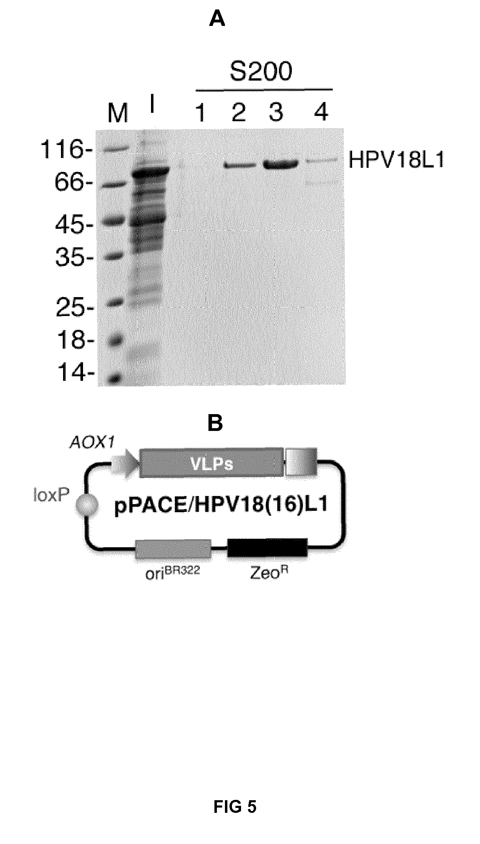

FIG 5: pPACE vector containing HPV18L1 protein (B) and SDS-PAGE electrophoretic profile of the HPV18L1 protein expressed in *P. pastoris* and purified by cation-exchange capture and S200 size exclusion chromatography (A). M: molecular ladder. I: Input (cell-free extract before purification). 1, 2, 3 and 4 correspond to different purification fractions over a size-exclusion chromatography column (Superdex 200, S200). HPV18L1 protein is mainly obtained in fraction 3.

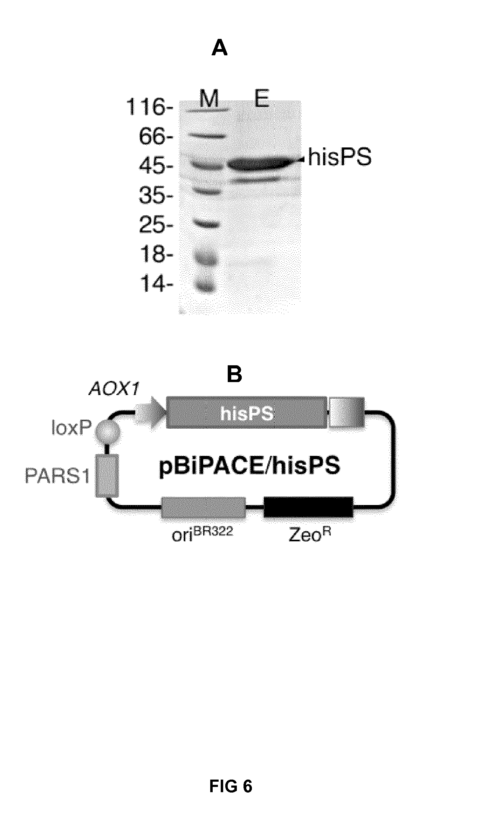

FIG 6: pBiPACE vector containing *Sch. pombe* PSAT gene (B) and SDS-PAGE electrophoretic profile of the PSAT protein expressed in *P. pastoris* and purified by Ni²⁺ IMAC capture and S200 size-exclusion chromatography (A). M: molecular ladder. E: expressed PSAT protein.

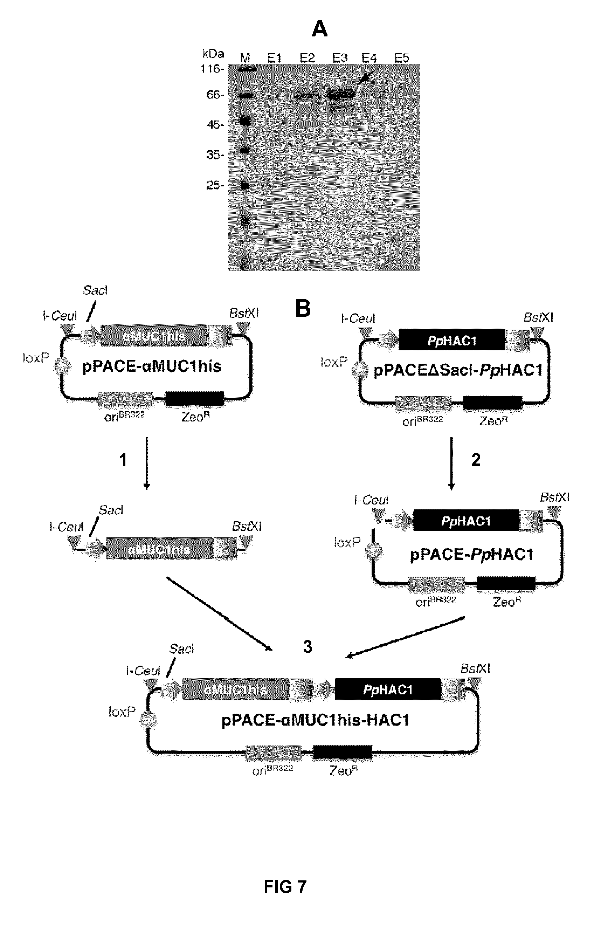

FIG 7: Construction of a vector containing multiple expression cassettes by HE/BstX1 assembly method using pPACE for co-expression of anti-MUC1 IIB6 scFv and *Pp*HAC1 (B). 1: I-CeuI/BstXI digestion; 2: I-CeuI digestion and Antarctic phosphatase treatment; 3: T4 DNA ligation. SDS-PAGE electrophoretic profile of the anti-MUC1 IIB6 protein co-expressed together with *Pp*HAC1 in *P. pastoris* and purified by Ni²⁺ IMAC capture and S200 size-exclusion chromatography (A). M: molecular ladder. E1-E5 correspond to different purification fractions. Anti-MUC1 IIB6 scFv protein is mainly obtained in fraction E3.

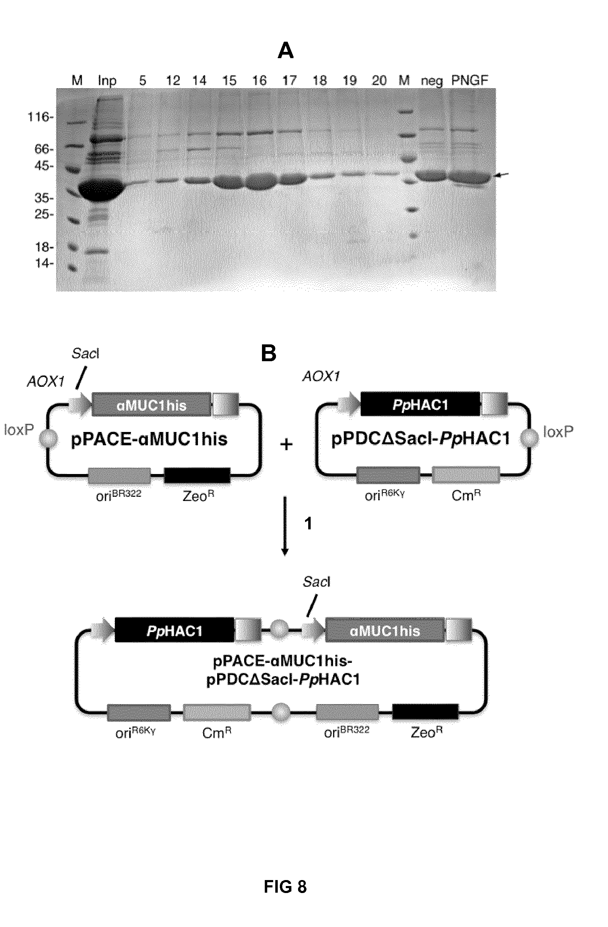

FIG 8: Construction of a vector containing multiple expression cassettes by Acceptor-donor fusion method using pPACE and pPDC for co-expression of anti-MUC1 IIB6 scFv and *Pp*HAC1 (B). 1: Cre-mediated recombination. SDS-PAGE electrophoretic profile of the anti-MUC1 IIB6 protein co-expressed together with *Pp*HAC1 in *P. pastoris* and purified by Ni²⁺ IMAC capture and S200 size exclusion chromatography (A). M: molecular ladder. 5-20 correspond to different purification fractions. anti-MUC1 IIB6 scFv protein is mainly obtained in fractions 15, 16 and 17. Fraction PNGF is fraction 16 treated with the deglycosylase PNGase F (NEB), and fraction neg represents the untreated control; the absence of a conspicuous shift in apparent molecular size indicates that overexpressed anti-MUC IIB6 scFv is very little glycosylated, if at all.

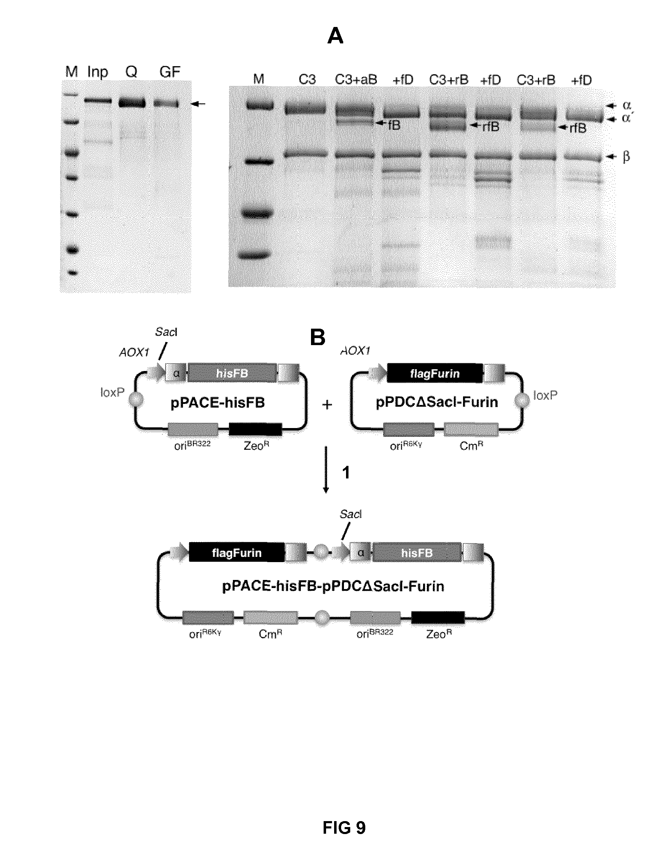

FIG 9: Construction of a vector containing multiple expression cassettes by Acceptor-donor fusion method using pPACE and pPDC for co-expression of human complement factor B, fB, and furin (B). 1: Cre-mediated recombination. SDS-PAGE electrophoretic profile of the fB protein co-expressed together with furin in *P. pastoris* and purified by anti-FLAG affinity capture and S200 size-exclusion chromatography (A). M: molecular ladder. Left-hand side SDS-PAGE gel: Inp, secreted recombinant fB; Q, fB purified by ionic exchange chromatography; GF, last purification step over a size-exclusion chromatography column (S200). Right-hand side SDS-PAGE gel: C3, complement factor C3, purified from plasma. C3+aB, C3 treated with fB obtained from plasma (aB stands for "authentic" fB). +fD, C3+aB+fD, where fD cleaves triggers the proteolytic cleavage of C3 into C3b and fB into fBa and fBb. C3+rB, as previous fraction C3+aB but using the recombinant fB produced in *P. pastoris.* +fD, C3+rB+fD, as previous fraction C3+aB+fD. The last two lanes (C3+rB and +fD) are as the previous two lanes, using recombinant fB from a second, independent expression and purification experiment, to demonstrate the reproducible authentic-like activity obtained from recombinant fB.

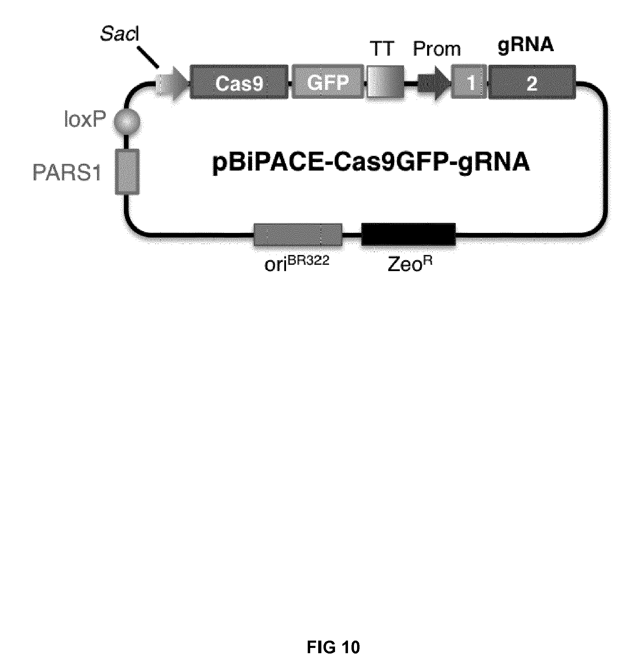

FIG 10: Construction of a pBiPACE vector containing a single synthetic DNA sequence by restriction-ligation into BstBI and Pmel sites, comprising two independently transcribed units, a Cas9GFP unit and a guide RNA (gRNA) unit under the control of a second promoter (Prom). The Cas9GFP unit comprises the AOX1 promoter (red arrow), Cas9GFP coding sequence, and the AOX1 terminator (TT). The gRNA unit is composed of a promoter (Prom) that drives the transcription of the ensuing two elements, a 19-21 bp target region (1) and the rest of the gRNA (2).

### DETAILED DESCRIPTION OF THE INVENTION

According to the present invention, the terms "nucleic acid" and "polynucleotide" are used interchangeably and refer to DNA, RNA or species containing one or more nucleotide analogues. Preferred nucleic acids or polynucleotides according to the present invention are DNA, most preferred double-stranded (ds) DNA.

In the sense of the present invention a "multiple integration element" (MIE) is understood as a nucleic acid comprising several sequence elements as defined in the first aspect of the invention that allows for cloning of one or more GOI and may be assembled by Cloning HE/BstXI-digested cassettes containing the gene of interest into the HE/BstXI sites of the MIE. The MIE of the invention is optimized for multiple gene expression in *P. pastoris.*

The term "expression cassette" is defined as known in the art of molecular biology as a nucleic acid sequence composed of one or more genes and the sequences controlling their expression (promoter, open reading frame and terminator). Additional sequence elements such as secretion signals, marker genes or purification tags may also be comprised within the expression cassette.

The term "vector" according to the present aspect of the invention is generally a nucleic acid suitable for integration of foreign genetic elements (in particular, one or more genes of interest) into host cells and which are suited for propagation and/or expression of the gene of interest. Appropriate vectors of the present invention are plasmids, expression vectors, transfer vectors, more preferred eukaryotic gene transfer vectors.

A "homing endonuclease" according to the present invention is a DNase specific for double-stranded DNA having a large, isometric recognition site of e.g. 12-40 base pairs or even more, preferably 20 to 30 base pairs.

The present invention provides a nucleic acid or polynucleotide comprising a MIE as defined in the first aspect of the invention (and Figure 1). The skilled person will understand that the MIE may comprise additional sequence elements, such as additional restriction sites, purification tags, etc. The MIE may also contain irrelevant sequences, spacers, etc.

The MIE contains a promoter and a terminator which are suitable for driving protein expression in *P. pastoris* hosts. Suitable promoters and terminators are selected from *AOX1, FLD1, FMD1, GAP* and *TEF1.* In order to satisfy the requirements of the multiprotein expression tools of the present invention, the promoter and terminator of the present MIE is modified with respect to the standard by destroying relevant restriction sites, namely, the HE, BstBI, Ncol, Pmel and BstXI restriction sites, if they are present in the sequence. The skilled person will understand that a suitable terminator sequence must accompany the selected promoter. The promoter contains a Kozak sequence functional in P. pastoris, preferably, the promoter has the following Kozak sequence: AAAACGATG.

In particular embodiments the promoter is AOX1 promoter and the terminator is AOX1 terminator. The AOX1 promoter is a strong, tightly regulated promoter which is strongly repressed when *P. pastoris* is grown on glucose, glycerol or ethanol. Upon depletion of these carbon sources, the promoter is de-repressed, but is fully induced only upon addition of methanol. This enables uncoupling the growth from the production phase, such that biomass is accumulated prior to protein expression. Therefore, cells are not stressed by the accumulation of recombinant protein during growth phase, and even the production of proteins that are toxic to *P. pastoris* is possible. Furthermore, it may be desirable to co-express helper proteins like chaperones at defined time points, for example, before the actual target protein is formed.

The AOX1 promoter of the present MIE is modified with respect to standard AOX1. In particular, Pmel and BstXI restriction sites are eliminated from the wild-type AOX1 promoter sequence. In particular embodiments, the AOX1 promoter in the MIE may contain additional modifications, for instance, other restriction sites may be destroyed. In a particular embodiment, the AOX1 promoter of the present MIE contains an additional modification consisting of the destruction of SacI site. The SacI site is usually used for plasmid linearization, and in this particular embodiment is deleted to allow for cassette multimerization without causing the multiplication of SacI sites.

In other embodiments, the AOX1 promoter sequence is further modified in order to enhance protein expression in the *P. pastoris* host. While optimizing AOX1 sequence, care needs to be taken not to generate undesired restriction sites that would be incompatible with other advantageous functionalities of the present multiprotein expression tool. In one particular embodiment, the sequence of the AOX1 promoter is SEQ ID NO: 1.

The MIE contains a homing endonuclease (HE) site and a specifically designed restriction enzyme site (BstXI) flanking the promoter and the terminator. Due to the length of HE recognition sequences it is highly unlikely that a corresponding site occurs in the nucleotide sequence of a gene or polygene (or any other nucleotide sequence of any origin) to be inserted into the constructs according to the present invention making this strategy particularly useful for cloning larger and/or many genes of interest.

A preferred HE site according to the present invention is a recognition sequence of a homing endonuclease that results in a 4 nucleotide overhang when cut by the respective homing endonuclease. Examples of such HE sites include, but are not limited to, recognition sequences of PI-SceI, I-CeuI, I-Ppol, I-Hmul I-Crel, I-Dmol, PI-Pful and I-Msol, PI-Pspl, I-Scel, other LAGLIDAG group members and variants thereof, SegH and Hef or other GIY-YIG homing endonucleases, I-Apell, I-Anil, Cytochrome b mRNA maturase bl3, PI- 77/1 and PI-Tfull, PI-Thyl and others. Corresponding enzymes are commercially available, e.g. from New England Biolabs Inc., Ipswich, MA, USA.

In particular embodiments, the HE is selected from the group consisting of a I-Ceul site, a PI-SceI site, a I-SceI site, a I-PpoI site, a I-CreI site and a I-DmoI site.

The BstXI restriction site in the MIE is complementary to the specific HE site. The recognition sequence of BstXI is defined as CCANNNNN'NTGG (SEQ ID NO: 2) (apostrophe marks position of phosphodiester link cleavage). The residues denoted as N are selected to match the overhangs generated by the HE cleavage. Table 1 shows HE/BstXI combinations appropriate for use in the MIE of the invention.

**Table 1. HE/BstXI combinations appropriate for the MIE**

| **HE** | **HE site** | **Corresponding BstXI site** |
|---|---|---|
| **I-CeuI** | TAACTATAACGGTCCTAAGGTAGCGAC (SEQ ID NO: 3) | ccat**CTAA**ttgg (SEQ ID NO: 4) |
| | CGCTACCTTAGGACCGTTATAGTTATG (SEQ ID NO: 5) | ggta**GATT**aacc (SEQ ID NO: 6) |
| **PI-SceI** | ATCTATGTCGGGTGCGGAGAAAGAGGTAATGAA ATGG (SEQ ID NO: 7) | ccat**GTGC**ctgg (SEQ ID NO: 8) |
| | TAGATACAGCCCACGCCTCTTTCTCCATTACTTT ACC (SEQ ID NO: 9) | ggta**CACG**gacc (SEQ ID NO: 10) |
| **PI-PspI** | TGGCAAACAGCTATTATGGGTATTATGGGT (SEQ ID NO: 11) | ccat**TTAT**ctgg (SEQ ID NO: 12) |
| | ACCGTTTGTCGATAATACCCATAATACCCA (SEQ ID NO: 13) | ggta**AATA**gacc (SEQ ID NO: 14) |
| **I-SceI** | TAGGG**ATAA**CAGGGTAAT (SEQ ID NO: 15) | ccat**ATAA**ctgg (SEQ ID NO: 16) |
| | ATCCC**TATT**GTCCCATTA (SEQ ID NO: 17) | ggta**TATT**gacc (SEQ ID NO: 18) |
| **I-PpoI** | CTCTC**TTAA**GGTAGC (SEQ ID NO: 19) | ccat**TTAA**ctgg (SEQ ID NO: 20) |
| | GAGAG**AATT**CCATCG (SEQ ID NO: 21) | ggta**AATT**gacc (SEQ ID NO: 22) |
| **I-CreI** | CTGGGTTCAAAACGTCGTGAGACAGTTTGG (SEQ ID NO: 23) | ccat**GTGA**ctgg (SEQ ID NO: 24) |
| | GACCCAAGTTTTGCAGCACTCTGTCAAACC (SEQ ID NO: 25) | ggta**CACT**gacc (SEQ ID NO: 26) |
| **I-DmoI** | ATGCCTTGCCGGGTAAGTTCCGGCGCGCAT (SEQ ID NO: 27) | ccat**GTAA**ctgg (SEQ ID NO: 28) |
| | TACGGAACGGCCCATTCAAGGCCGCGCGTA (SEQ ID NO: 29) | ggta**CATT**gacc (SEQ ID NO: 30) |

In one embodiment the invention provides a MIE wherein the HE/BstXI restriction sites are selected from the following HE/BstXI pairs: SEQ ID NO: 3/SEQ ID NO: 4, SEQ ID NO: 7/SEQ ID NO: 8, SEQ ID NO: 11/SEQ ID NO: 12, SEQ ID NO: 15/SEQ ID NO: 16, SEQ ID NO: 19/SEQ ID NO: 20, SEQ ID NO: 23/SEQ ID NO: 24, SEQ ID NO: 27/SEQ ID NO: 28, or their complementary sequences.

One of the main advantages of using *P. pastoris* as a protein production host is its ability to secrete high titers of properly folded, post-translationally processed and active recombinant proteins into the culture media. As a rule of thumb, proteins secreted in their native hosts will also be secreted in *P*. *pastoris.* However, quite often it is convenient to achieve secretion of expressed proteins which are not naturally secreted. For this aim, the MIE of the invention includes a sequence encoding for a secretion signal peptide. In a particular embodiment, the secretion signal peptide is the *S. cerevisiae* α-mating factor secretion signal (alpha-MF). As explained above, the particular design of the MIE provides a dual functionality: by simply selecting the appropriate digestion strategy, the secretion signal may be fused with the gene of interest, or not, depending on whether the user desires to enhance secretion of the protein of interest or if intracellular expression is preferred.

Further, in a particular embodiment of the invention the alpha-MF secretion signal sequence has been modified to enhance its secretory potential in *P*. *pastoris.* In a particular embodiment, the sequence for the alpha-MF secretion signal is SEQ ID NO: 50.

The MIE of the invention also contains the sequence encoding for a selection marker gene, which allows for rapid selection of clones containing the gene(s) of interest. Non-limiting selection marker genes appropriate for use in the MIE of the invention are lacZ, luciferase, β-GAL, CAT and fluorescent encoding protein genes such as GFP, BFP, YFP, CFP and their variants. In a particular embodiment, the selection marker gene is LacZ.

The MIE also contains a sequence encoding a detection and/or purification polypeptide. A "purification polypeptide" is, as generally known in the art, a peptide or protein useful for isolating or purifying the expressed protein. Said polypeptides often have affinity for a particular binding molecule, a property that is used for the purification/isolation. Likewise, a "detection polypeptide" is a peptide or protein useful for easily detecting the expressed protein. Sometimes the particular polypeptide serves both detection and purification purposes. Appropriate, non-limiting, purification and/or isolation polypeptides for the present MIE are: poly-histidine (6-20 histidine residues), FLAG-tag, StrepII-tag, HA epitope, c-Myc epitope, V5 epitope, calmodulin-binding peptide (CBP), enhanced green fluorescent protein (EGFP), enhanced yellow fluorescent protein (EYGP), Rudolph red fluorescent protein (RFP), monomeric Cherry fluorescent protein (mCherry), cyan fluorescent protein (CFP); these tags may be preceded by a protease cleavage sequence selected from the following: tobacco etch virus (TEV), 3C, enterokinase or carboxypeptidase A (CPA). In a particular embodiment the purification polypeptide is a poly-histidine sequence (KHis tag) comprising from 6 to 20 histidine residues following the Pmel restriction site. In particular, the KHis tag comprises from 6 to 16, or from 7 to 15, or from 8 to 12, for example 9, 10 or 11 histidine residues. Said KHis tag allows the GOI to be expressed with a C-terminal purification histidine tail.

The MIE of the invention may contain additional sequence elements. For instance, it may comprise further restriction sites and/or further detection and/or purification polypeptides. In one embodiment, the MIE additionally comprises a KpnI and/or MscI and/or Xbal restriction sites. In another embodiment, the MIE comprises a second KHis tag, wherein the second KHis tag comprises from 4 to 20 histidine residues. In particular, the second KHis tag comprises from 5 to 10, for example 6, 7, 8 or 9 histidine residues. In a particular embodiment, the MIE comprises the sequence elements as represented in Figure 2. Further particular embodiments define a MIE of SEQ ID NO: 31 and SEQ ID NO: 32.

In a particular embodiment the invention provides the MIE contains AOX1 promoter and terminator sequences devoid of Pmel and BstXI restriction sites, a BstBI restriction site, a secretion signal sequence, an Ncol restriction site, a selection marker gene, a Pmel restriction site, a sequence encoding for a KHis tag comprising from 6 to 20 histidine residues and a BstXI restriction site. In another particular embodiment the MIE contains AOX1 promoter and terminator sequences devoid of Pmel and BstXI restriction sites, a BstBI restriction site, an alpha-MF secretion signal sequence, an Ncol restriction site, a selection marker gene, a Pmel restriction site, a sequence encoding for a KHis tag comprising from 6 to 20 histidine residues and a BstXI restriction site. In another particular embodiment the MIE contains AOX1 promoter and terminator sequences devoid of Pmel and BstXI restriction sites, a BstBI restriction site, an alpha-MF secretion signal sequence, an Ncol restriction site, a lacZ selection marker gene, a Pmel restriction site, a sequence encoding for a KHis tag comprising from 6 to 20 histidine residues and a BstXI restriction site. Further particular embodiments provide MIEs where the AOX1 sequence is SEQ ID NO: 1 and the alpha-MF sequence is SEQ ID NO: 50.

The invention also refers to an expression cassette obtained by inserting at least one GOI into the MIE. It is noted that the expression cassette of the invention may contain more than one, particularly from 1 to 10 GOI. The one or more GOI may be inserted into the MIE of the invention by methods known to the skilled person, e.g. by restriction enzyme digestion/ligation via compatible sites within the MIE or by recombination, preferably using ligase-independent methods, e.g. the SLIC method. In one embodiment, the GOI may be inserted between the Ncol and Pmel sites and the resulting expression cassette will then include the signal sequence, such that said GOI will be expressed as a secretion peptide(s). The GOI may be alternatively inserted between the BstBI and Pmel sites and will then be expressed as an intracellular peptide. The resulting expression cassettes usually contain a C-terminal KHis tag. Alternative embodiments provide for expression cassettes comprising a N-terminal KHis tag and/or further N- and C- terminal fusions. N-terminal fusions can be added to the expression cassette by cloning the GOI into NcoI/KpnI restricted MIE, and C-terminal fusions can be added similarly by cloning into PmeI/MscI restricted MIE. The Xbal site in the MIE can be used for the insertion of e.g., GFP and other C-terminal fusion tags.

According to one aspect, the invention provides a vector comprising a MIE or an expression cassette as defined above. In particular embodiments of the present invention, the vector of the invention comprising the above defined MIE or expression cassette additionally comprises at least one site for integration of the nucleic acid into a vector or host cell.

Particularly preferred integration sites that may be incorporated into the present vector can be selected from site-specific recombinases recognition sites, in particular LoxP site or short flippase recognition target (FRT) site. In a particular embodiment, the vector of the invention comprises at least one LoxP site or FRT site for integration by Cre-loxP recombination or Flp-FRT recombination, respectively.

In particular embodiments of the present invention, the vector as described herein additionally contains sequences encoding for one or more resistance markers for selecting against otherwise toxic substances. Preferred examples of resistance markers useful in the context of the present invention include, but are not limited to, antibiotics such as zeocin, phleomycin, ampicillin, chloramphenicol, gentamycin, spectinomycin, blasticidin, hygromycin and kanamycin. In a particular embodiment, the antibiotic resistance marker is zeocin. In another particular embodiment, the sequence encoding for the zeocin resistance marker is optimized for improved zeocin expression in *P*. *pastoris* host. In another particular embodiment the sequence encoding for the zeocin resistance marker has SEQ ID NO: 33.

In addition to all the above, vectors of the invention usually comprise an origin of replication (ori). Examples of suitable oris are BR322, ColE1, as well as conditional origins of replication such as OriV and R6Kγ, the latter being a preferred conditional origin of replication which makes the propagation of the vector of the present application dependent on the pir gene in a prokaryotic host. OriV makes the propagation of the vector of the present application dependent on the trfA gene in a prokaryotic host.

One of the most imposing bottlenecks in the *P. pastoris* host is that genome integration is a prerequisite for expression, since very few autonomously replicating sequences are available. Genome integration requires large amounts of restriction enzyme-linearized plasmid per transformation (typically several micrograms pure plasmid per electroporation) and lengthy procedures for selection of high-copy integrands. The present invention provides for vectors comprising the PARS1 sequence that circumvent the need for genome integration of the expression cassette thereby allowing expression tests to be run both from a self-replicating plasmid (like in conventional bacterial expression systems) and from a genome-integrated copy of the linearized plasmid (for stable protein production). Within the vector of the invention, PARS1 sequence is preferably inserted upstream the site for integration (for example upstream a IoxP site).

In some embodiments, the vector comprises a MIE as defined above, at least one site for integration, a resistance marker and an origin of replication. In other embodiments, the vector additionally comprises a PARS1 sequence. In one embodiment, said origin of replication is BR322, said resistance marker is zeocin and said MIE comprises I-CeuI restriction site, for example a MIE of SEQ ID NO: 31. In another embodiment said origin of replication is BR322, said resistance marker is zeocin, said MIE comprises I-CeuI restriction site, for instance a MIE of SEQ ID NO: 31, and the vector additionally comprises a PARS1 sequence upstream from the site for integration. In another embodiment said origin of replication is a conditional origin of replication selected from OriV and R6Kγ, said resistance marker is an antibiotic selected from the group consisting of ampicillin, chloramphenicol, gentamycin, spectinomycin, blasticidin, hygromycin and kanamycin, and said MIE comprises PI-SceI restriction site and AOX1 promoter lacking also SacI restriction site, for example a MIE of SEQ ID NO: 32.

Further particular embodiments of the present invention are vectors containing more than one of the sequence elements as defined above. For example, the vector may contain more than one recombination sequence for a site specific recombinase, e.g. 1 to 6, for example 2, 3, 4 or 5 of such recognition sequences, preferably 1 to 6, for example 2, 3, 4 or 5 IoxP sites.

Particular embodiments of the invention provide a vector consisting of a sequence selected from the group consisting of SEQ ID NO: 34 to SEQ ID NO: 38.

In another aspect, the present invention is directed to a host cell containing the nucleic acid of the invention and/or the vector of the present invention. The host cells may be prokaryotic or eukaryotic. Prokaryotic hosts according to the present invention include bacteria, in particular *E. coli* such as commercially available strains like TOP10, DH5α, HB101 etc., suitable for the propagation of the above vectors. Eukaryotic host cells are preferably *P*. *pastoris* strains for expression of the GOI. Virtually any *P. pastoris* strain can be used with the vectors according to the invention. The only requirement when zeocin-resistance cassette (Sh ble) is the resistance marker in the vector is that the Zeo resistance marker has not been used in the recipient strain, which covers most or all *P. pastoris* strains of industrial application. However, if the Zeo resistance marker had already been used in the recipient strain, the plasmid fusion mechanism allows to circumvent this limitation by adding any genomic locus suitable for integration into the multigene construct via the donor vectors (see below), therefore opening up the whole *P. pastoris* genome for integration; e.g., the gene for tubulin could be cloned into a Donor vector (e.g. pPDC, see below) and fused with the Acceptor vector carrying a GOI (e.g. pPACE--GOI), the resultant construct could be linearized at the ADE2 gene site therefore directing the plasmid toward the ADE2 locus exploited in the commercially available *P*. *pastoris* PichiaPink strains (Invitrogen). Otherwise, the present system does not impose any undue restraints on the yeast strain's genome and/or phenotype.

The person skilled in the art is readily able to select appropriate vector construct/host cell pairs for appropriate propagation and/or transfer of the nucleic acid elements according to the present invention into a suitable host. Specific methods for introducing appropriate vector elements and vectors into appropriate host cells are equally known to the art (Green M and Sambrook J (2012), *infra).*

The present invention also relates to a method for producing a vector containing multiple expression cassettes as defined above. According to preferred embodiments of the present invention, it is possible to insert one or more genes into the vectors of the invention by methods known to the skilled person. In certain embodiments, the vector of the invention comprises from 1 to 10 expression cassettes each containing a GOI, cloned in series by repeated use of the HE/BstXI multimerization cassette. In other embodiments, 1-10 GOI can be cloned into several different vectors of which one must be an acceptor vector, and then fused together by Cre-IoxP recombination into a larger plasmid containing the complete set of GOIs cloned into the individual plasmids as explained below. In a further embodiment pertaining only to proteins targeted to the secretory pathway of *P. pastoris,* the person skilled in the art may choose to concatenate several GOI (typically, between 1-5) into a polygene with a Kex2 or a furin cleavage sequence between the 3' end of a GOI and the 5' of the following GOI. Post-translational proteolytic processing of the polyprotein inside the endoplasmic reticulum should split the polyprotein into the constituent proteins. Thus in other embodiments the vector of the invention comprises from 1 to 10 expression cassettes, preferably from 1 to 5 expression cassettes, wherein each cassette may contain either 1 GOI or 1-5 concatenated GOI.

As already mentioned, the vectors of the invention may contain a site for integration into another vector or host cell. In preferred embodiments, this site for integration is a site-specific recombinase recognition sequence, such as a IoxP site or FRT site. This has the advantage that said vectors, which may contain one or several expression cassettes obtained as described above, can be assembled into a fusion vector comprising several vector entities separated from each other by a site-specific recombination site, wherein each vector entity contains an individual resistance marker gene different from the resistance marker genes of the other vector entities.

A "vector entity" as used herein refers to a vector according to the invention comprising at least one expression cassette, at least one site for integration, a resistance marker, and which is suitable for propagation. Preferred examples of the vector entities are those defined by SEQ ID NO: 34 to SEQ ID NO: 38.

Thus, the present invention further provides a method for assembling n vector entities into 1 to (n-1) fusion vectors wherein said fusion vector(s) contain(s) 2 to n of said vector entities comprising the steps of:
(1) contacting n vector entities each containing a site-specific recombination site and an individual resistance marker different from the resistance markers of the other vector entities so as to generate a mixture of fusions of the vector entities comprising 2 to n of said vector entities;
(2) transforming said mixture into host cells;
(3) culturing one or more sample(s) of the transformed cells in the presence of the appropriate combination of antibiotics for selecting a desired fusion vector containing 2 to n vector entities;
(4) obtaining n single clones of transformed cells from the culture obtained in step (3) in which these were viable in the presence of the respective combination of antibiotics; and
(5) culturing n samples of each of said n single clones in the presence of each of n antibiotics specific for the n individual resistance markers present in said n vector entities, wherein n is an integer of at least 2 to 9, for example 3, 4, 5, 6, 7 or 8.

The term "culturing" means that the cells are incubated under the appropriate conditions for viability of the host cells.

It is clear for the skilled person that the number of vector entities to be assembled into a fusion vector according to the present invention is generally not specifically limited as long as a corresponding number of resistance markers are available. Examples of resistance markers useful in the context of this aspect of the present invention are as already defined above. Further details regarding the method for assembly of fusion vectors are disclosed in the examples below.

The principle underlying the above-described method for assembling a fusion vector, i.e. the equilibrium of educts and products in recombination reactions, can equally be applied to the disassembly of fusion vectors. Therefore, the invention further provides a method of disassembling a fusion vector containing n vector entities into one or more desired fusion vectors selected from the group consisting of fusion vectors containing 2 to (n-1) vector entities or into one or more desired single vector entities, wherein in said fusion vector containing n vector entities said n vector entities are separated from each other by n site-specific recombination sites and each vector entity contains an individual resistance marker different from the resistance markers of the other vector entities, comprising the steps of: (A) contacting the fusion vector containing n vector entities with a recombinase specific for said site-specific recombination sites in order to generate a mixture of fusion vectors comprising 2 to (n-1) of said vector entities and single vector entities; (B) transforming said mixture into host cells; and (C) culturing one or more sample(s) of the transformed cells in the presence of (C1) an appropriate combination of antibiotics for selecting one or more desired fusion vector(s) containing 2 to (n-1) vector entities; and/or (C2) a single appropriate antibiotic for selecting a desired single vector entity; (D) obtaining n single clones of transformed cells from the sample of the transformed cells in which these were viable in the presence of the respective antibiotic or combination of antibiotics, respectively; and (E) culturing n samples of each of said n single clones in the presence of each of n antibiotics specific for the n individual resistance markers present in said n vector entities; wherein n is as defined above.

According to a particular embodiment of the invention, the above-defined vector entities contain at least one multiple expression cassette. Each expression cassette present in the vector entity may contain one or more genes of interest. Preferred embodiments of the above fusion vector contain LoxP sites and Cre is the corresponding recombinase enzyme for vector assembly.

According to a preferred embodiment of the present vector assembly method, (n-1) of the vector entities to be fused each contains a further selectable marker different from the (antibiotic) resistance marker. Such vector entities are hereinafter referred to as "Donor" vectors, since, when fused to a vector entity which does not contain said selectable marker different from the resistance marker (hereinafter referred to as "Acceptor"), in a fusion between the Donor(s) and the Acceptor, said Donor(s) provide host cells with a phenotype that allows only the propagation of Acceptor- Donor fusions but not Donor-Donor fusions. Preferred examples of such a selectable marker are conditional origins of replication making the propagation of the Donor dependent on a specific genetic background as defined above. In a particular embodiment, the selectable marker is R6Kγ ori making the propagation of the Donor dependent on the presence of the pir gene in a bacterial host such as *E. coli.* In this case, the mixture obtained in step (1) of the above vector assembly method is transformed into bacterial cells lacking the pir gene (such *E. coli* strains TOP10, DH5α, HB101 or other commercially available pir+ cells). Donor vectors according to the present embodiment may contain AOX1 promoter lacking SacI restriction site.

The vectors of the invention may be included in a kit for cloning and/or expression of proteins. One embodiment is directed to a kit for cloning and/or expression of proteins comprising at least one, for example from 1 to 10, vectors as disclosed herein. Preferably the kit comprises at least one vector, i.e. from 1 to 10 vectors, comprising a MIE or an expression cassette as defined above, an origin of replication such as BR322, ColE1, OriV or R6Kγ, a resistance marker, such as zeocin, ampicillin, chloramphenicol, gentamycin, spectinomycin, and kanamycin resistance markers, at least one site for integration such as a LoxP site or FRT site, and optionally a PARS1 sequence. In addition to the vectors as defined above, the kit of the invention may contain a host cell suitable for propagation of said vector(s). The kits may also contain *P. pastoris* host cells suitable for expression.

In one embodiment the kit comprises: (i) at least one (acceptor) vector comprising a MIE or expression cassette which contains I-CeuI as HE restriction site, a BR322 origin of replication, an antibiotic resistance marker, such as ampicillin, chloramphenicol, gentamycin, spectinomycin, blasticidin, hygromycin or kanamycin, a site for integration and, optionally, a PARS1 sequence; and/or (ii) at least one (donor) vector comprising a MIE which contains PI-SceI as HE restriction site, a conditional origin of replication, such as OriV or R6Kγ, an antibiotic resistance marker, such as ampicillin, chloramphenicol, gentamycin, spectinomycin or kanamycin and a site for integration. Preferably each vector included in the kit has a resistance marker different to the resistance marker of the other vectors. In a particular embodiment, the kit comprises: (i) at least one vector selected from the (acceptor) vectors with SEQ ID NO: 34 and SEQ ID NO: 35; and/or (ii) at least one (donor) vector selected from the vectors with SEQ ID NO: 36, SEQ ID NO: 37 and SEQ ID NO: 38. In particular embodiments the kit comprises both acceptor and donor vectors as defined above. In addition to the vectors as defined above, the kit of the invention may contain a host cell suitable for propagation of said vector(s). The kits may also contain *P. pastoris* host cells suitable for expression.

The kit according to the invention, as defined above, is particularly suitable for cloning and/or expression of multiprotein complexes in *P. pastoris.* In a particular embodiment, the kit comprises acceptor and/or donor vectors as defined above, host cells suitable for propagation of the vectors and expression of the GOI and instructions for obtaining multiple-gene constructs and expression of the same to provide for the simultaneous expression of multiple proteins.

In another embodiment, the kit of the invention may comprise at least one vector comprising an expression cassette which contains one or more "helper genes". In particular embodiments, said vector is of the kind of a donor vector as defined above, and said helper gene encodes for a protein serving a particular purpose related to the expression of the GOI, e.g., chaperons, activator, secretion enhancer, ribosome recycling factor, ribosome, wide-spectrum phosphatases, post-translational modifiers, etc. In the sense of the present invention, the vectors containing these helper genes are termed "helper vectors". This helper vectors can be incorporated into to the fusion vector following the vector assembly method described above and, when transformed into the appropriate *P. pastoris* host, the helper gene will be co-expressed with the GOI resulting in a positive impact in the yield or quality (or both) of the heterologous expression. Non-limiting examples of helper genes are listed in table 2.

**Table 2. Helper genes**

| **Purpose** | **Helper gene** |
|---|---|
| *P. pastoris* secretion | *Sc*HAC1p, *Pp*HAC1p, *Pp*PDI, *Pp*BIP/Kar2 |
| Secretion of mammalian proteins | Furin |
| General chaperones | Hsp40, Hsp90, Hsc70, HOP/Stip1 |
| Genome targeting | Tubulin |

| | |
|---|---|
| Sc = S. cerevisiae Pp = P. pastoris | |

Herein follows a complete description of preferred MIE, vectors, kits and methods of the present invention, all encompassed within a multi-protein expression system termed "MultiPichia". General experimental procedures to generate multigene expression constructs are also provided.

### A. MultiPichia System

### A.1 MultiPichia vectors

The present invention provides as preferred exemplary embodiments small de novo designed vectors, which are called "Acceptor", and "Donor" vectors (for plasmid maps see figure 3). Acceptor vectors for expression of proteins in *P*. *pastoris* [e.g. pPACE (SEQ ID NO: 34) and pBiPACE(SEQ ID NO: 35)] contain origins of replication derived from ColE1 (pMB1) and resistance marker (zeocin, Zeo). Donor vectors [e.g. pPDK (SEQ ID NO: 36), pPDC (SEQ ID NO: 37) and pPDS (SEQ ID NO: 38)] contain conditional origins of replication derived from R6Kγ, which make their propagation dependent on hosts expressing the pir gene. Donor vectors contain resistance markers kanamycin (Kn), chloramphenicol (Cm), and spectinomycin (Sp). Preferably, one or two Donor vectors are used in conjunction with one Acceptor vector.

All Donor and Acceptor vectors according the present example contain a LoxP imperfect inverted repeat and, in addition, a multiple integration element (MIE), which follows the basic layout outlined above. In particular, the MultiPichia MIE used in the present example contains, in addition to the HE and BstXI restriction sites, (1) the modified AOX1 promoter-terminator sequences, (2) alpha-MF secretion signal sequence flanked by restriction enzyme sites (5' BstBI and 3' Ncol); (3) a prokaryotic promoter upstream of the LacZα selection marker gene to support blue-white selection; (4) in the latter sequence, there are restriction sites designed to insert N-terminal (KpnI) and / or C-terminal fusion sequences (Xbal, Mscl) (e.g., StrepII, calmodulin binding peptide); and (5) an optional C-terminal lysine-decahistidine (Cter-KH10).

Obviously, due to the presence of the HE and BstXI sites, the expression cassettes can be easily interchanged between the vectors, either "empty" (LacZα) or with an inserted gene. This can be done by restriction ligation or by restriction enzyme / ligase independent methods (e.g., SLIC). Therefore, versions can be created which contain other promoter-terminator cassettes (e.g., GADH, TEF1) with any one of the resistance markers or others (e.g., blasticidin, hygromycin). The HE/BstXI site combinations can be used to multiply expression cassettes or to introduce alternative or multiple promoter sequences.

All Donor vectors contain a LoxP imperfect inverted repeat. This can be used for LoxP-mediated constructions (and deconstructions) of Acceptor / Donor multifusions as described above.

### B.2 Multiple integration element (MIE)

The MIE allows for several approaches for multigene assembly (see Section C below). Multiple genes can be inserted into the MIE of any one of the vectors by ligation-dependent as well as seamless cloning (e.g., BD InFusion, SLIC). In every case, the vector needs to be linearized, either by restriction digestion or by PCR with suitably chosen primers. Use of ultrahigh-fidelity polymerases such as Phusion (NEB) is preferred. The map of the present MIE is shown in Figure 1. DNA sequences for MIE are provided as SEQ ID NO. 31 (containing I-CeuI) and SEQ ID NO: 32 (containing PI-SceI). A map of the MIE used in the MultiPichia system is provided in Figure 2.

### A.3 Tags, promoters and terminators

The AOX1 promoter is a strong inducible (with methanol) promoter that can be utilized in all strains of *P. pastoris.* Other alternative promoters are available (e.g., GADPH) or can be easily introduced should it be required.

In available vectors for *P. pastoris* a choice has to be made between cloning the gene insert in a vector for intracellular expression or for secretion. In the MultiPichia system, the same vectors can be used for both purposes because a α-MS sequence is always present in the vectors and the choice of localization is facilitated by the presence of a set of specific restriction sites. As exemplified in Figure 1, linearizing the vector with BstBI-PmeI allows intracellular expression by removing the αMF leader sequence, whereas linearizing with Ncol-Pmel allows secretion expression by ensuring the correct reading frame between the αMF and the gene of interest sequence. In the former case, the Kozak sequence that lies 5' to the αMF has to be rebuilt after BstBI digestion since it is partially destroyed, which is conveniently done by the appropriate choice of PCR primers (infra).

The complete multiple integration element (MIE) in MultiPichia Donor and

Acceptor vectors, including promoters and terminators, excluding the HE and BstXI sites, can be exchanged with an expression cassette of choice by using restriction-ligation cloning with appropriate enzymes (e.g., EcoRI-NcoI, HE-Ncol). For example, the AOX1 promoter in pPACE can be replaced with the GAPDH promoter (pPACEGAP) for constitutive expression.

The MultiPichia Acceptor and Donor vectors provide optional N-terminal hexahistidine-3C tag (His6-3C) and / or C-terminal lysine-decahistidine (Cter-KH10) tags as well as a standardized mechanism for the introduction of further N- and C-terminal fusions. N-terminal fusions can be added to any one vector by cloning into NcoI-KpnI, and C-terminal fusions can be added similarly by cloning into PmeI-MscI the desired cassette. The MultiPichia vectors also include an Xbal site between Pmel and Mscl, which can be used for the insertion of e.g., GFP and other C-terminal fusion tags.

### A.4 Complex expression

For expression in *P. pastoris,* the MultiPichia multigene expression fusion vector is transformed into the appropriate expression host of choice. The present exemplary vectors (AOX1 promoter elements) can be used with all currently available *P. pastoris* strains. In the current implementation, the Acceptor vector carries a Zeocin resistance marker, hence if the multigene fusion is to be transformed into a *P. pastoris* strain harboring other Zeocin markers, alternative versions of the MultiPichia Acceptor vector with an alternative resistance that resolved the conflict should be prepared (e.g., blasticidin, hygromycin, G418). This can be easily achieved by PCR amplifying the vector less the resistance marker, and combine the resulting fragment with a PCR amplified resistance marker by SLIC or blunt-end ligation (using 5'-phosphorylated primers). All resistance markers present in the MultiPichia Donor vectors are intended for selection in *E. coli* and should not interfere with yeast expression.

Donor vectors of the present example depend for their propagation by *E. coli* of the pir gene product by virtue of the R6Kγ conditional replication origin. These vectors cannot be used as such for expression in *P. pastoris* since they lack yeast-compatible resistance markers. However, the expression performance of the gene inserts present in Donor vectors can be accomplished by fusion with an empty Acceptor vector, which would act as a vehicle into the expression host.

The present MultiPichia system for expression in *P. pastoris* contains two Acceptor vectors, pPACE and pBiPACE, which are identical except for the presence of a PARS1 (*Pichia* autonomous replication sequence 1) (Figure 3). pPACE requires genome integration and selection and leads to stable protein producing strains, whereas transformation of *P. pastoris* cells with pBiPACE leads to a transient phase whereby the vector stays as an episomal element. The advantage of pBiPACE over pPACE is that expression tests can be performed in a rapid and economic fashion while evaluating the relative performance of multiple constructs. Once a particular construct is selected for scaling up, linearization of pBiPACE or the use of pPACE will trigger genome integration of the expression cassette and a more robust expression strain. These two approaches are outlined in Figure 4.

### B. Procedures

### B.1 Cloning into MultiPichia vectors

All Donor and Acceptors of the preferred embodiment for expression in *P*. *pastoris* contain an identical MIE with exception of the homing endonuclease site / BstXI tandem spanning the MIE (Figures 1 and 2). The MIE is designed for sequence and ligation-independent gene insertion methods. In addition, the MIE contains two sets of unique restriction sites (Ncol / KpnI and Pmel / Mscl) that can be used as a classical multiple cloning site for conventional gene insertion by restriction / ligation. For automated applications, insertion of genes of interest is preferably done by recombination approaches (e.g., SLIC, BD InFusion).

### B.1.1 Single gene insertion into the MIE by SLIC

There are several standard methods for insertion of single genes into vectors that have been published or commercialized (e.g., BD InFusion, SLIC). All these methods rely on the 3' to 5' exonuclease activity of DNA polymerases. In the absence of dNTPs, 5' overhangs are created starting from blunt ends or overhangs by digestion from the 3' end. If two DNA fragments contain the same ca. 20-50 bp sequence at their ends, complementary overhangs can be produced that can anneal to produce a nicked double-stranded DNA joint fragment. This phenomenon can be exploited for ligation-independent combination of two or more DNA fragments containing terminal homologous sequences.

If T4 DNA polymerase or other commercially available DNA polymerases (e.g., BD InFusion) are used, seamless cloning can be carried out in a sequence and ligation-independent fashion and detailed protocols are available for the skilled person. This is advantageous for multiprotein expression since the longer constructs have a higher chance of containing restriction enzyme (either naturally present or introduced by unwanted mutagenesis) sites that would be incompatible with the conventional restriction / ligation cloning.

In the present invention, the SLIC strategy was tailored for inserting encoding DNAs amplified by Phusion polymerase into the MultiPichia Donor and Acceptor vectors. The streamlined, automatable protocol that is recommended for MultiPichia takes care of seamless integration of single genes into the expression cassettes and of concatamerization of expression cassettes into multigene constructs.

The following Protocol 1 represents an adaptation of the method described by Bieniossek et al. (2009, *infra)* and an improved process based on the method described by Li et al. (2007, *infra).* Protocol 1 is designed for manual operation although it is easily automatable to work in robotics platforms. Other cloning strategies can be used (e.g., BD InFusion) following the manufacturer's recommendations.

**Protocol 1:** Single gene insertion by SLIC.

Reagents required:
Phusion polymerase (2 U/ul)
5X HF / GC Buffer for Phusion polymerase
dNTP mix (10 mM)
T4 DNA polymerase (3 U/ul) (and 10X buffer)
DpnI (20 U/ul)
*E. coli* competent cells
100 mM DTT, 2 M urea, 0.5 M EDTA
1 M IPTG, 100 mg/ml X-Gal or Bluo-Gal
Antibiotics

### Step 1: Primer design

Primers for the SLIC procedure are designed such that their 5' sequences provide homology regions complementary to the MultiPichia vectors. When treated with T4 DNA polymerase in the absence of dNTPs, long sticky ends would be generated.

Primers for the insert contain a DNA sequence corresponding to this homology region ("Adaptor sequence"), followed by a sequence that specifically anneals to the insert to be amplified ("Insert specific sequence"). Useful sequences used with the MultiPichia system are listed below (Table 3).

The "insert specific sequence" can be located upstream of a Kozak sequence if the gene of interest (GOI) is amplified from a vector that already contains expression elements and the MultiPichia vectors are linearized to eliminate the secretion peptide leader sequence. For secretion expression there is no need to include a Kozak sequence in the primers since it is already present in the vector. Only when intracellular expression is desired a Kozak sequence must be included in the forward primer so that a ribosome-binding site is provided in the final construct. A suitable Kozak sequence is contained in AAACGATG (underlined).

A choice can be made as to the inclusion of a stop codon (e.g., TAA) in the reverse primer between the "insert specific sequence" and the "vector adaptor sequence". When a stop codon is included, a 3' untagged protein product will be expressed; otherwise, the vector-encoding 3' fusion will be appended to the expressed protein (e.g., in the default vector is a KHis10 tag).

Primers for PCR linearization of the vector backbone are designed to be complementary to the two adaptor sequences present in the primer pair chosen for insert amplification.

### Step 2: PCR amplification of insert and vector

### Step 3: DpnI treatment of PCR products (optional)

### Step 4: Purification of PCR products

Steps 2-4 can be performed as per standard molecular biology techniques, e.g., Bieniossek et al. (2009, *supra).*

### Step 5: T4 DNA polymerase exonuclease treatment

### Step 6: Mixing and annealing

The procedure for Steps 5 and 6 are available for a skilled person (e.g., Bieniossek et al. (2009, *supra*)). For the MultiPichia system the present invention incorporates a modification that increases the speed and ease of the method and which consists in carrying out both steps with premixed components (a "one-pot" reaction):
Reactions are prepared in 20 ul containing vector and insert in a 1:2 molar ratio (eluted in Step 4):

| | |
|---|---|
| 10X T4 DNA polymerase buffer | 2 ul |
| 100 mM DTT | 1 ul |
| 2 M urea | 2 ul |
| DNA (vector and insert) | 14 ul |
| T4 DNA polymerase | 1 ul |

Reactions are then carried out as follows; this sequence can be easily programmed in a thermocycler:

| | |
|---|---|
| Incubation: | 23 °C for 20 min |
| Arrest: | Addition of 1 ul 0.5 M EDTA |
| Inactivation: | 75 °C for 20 min |
| Annealing: | 65 °C for 10 min, 37 °C for 30 min |
| Cooling: | 4 °C, on hold |

### Step 7: Transformation

Reaction mixtures (from Step 6) can be transformed into *E. coli* competent cells following standard transformation procedures.

Reactions for pPACE and pBiPACE plasmids are transformed as pACE and pACE2 of the ACEMBL system, except that 25 ug/ml Zeocin is used for selection. Since the MIE of the "empty" vectors encode the LacZα fragment, plating on Luria-Bertani medium with 1 mM IPTG and 0.1 mg/ml X-Gal or Bluo-Gal should result in white (recombinant) and / or blue (parental) colonies.

Reactions for Donor plasmids are transformed in *pir*+ *E. coli* cells as the Donor derivatives of the ACEMBL system, with the same antibiotics. For the Donor derivatives pPDC, pPDK, and pPDS, it is highly recommended that electrocompetent cells be used. Since the MIE of the "empty" vectors encode the LacZα fragment, plating on Luria-Bertani medium with 1 mM IPTG and 0.1 mg/ml X-Gal or Bluo-Gal should result in white (recombinant) and / or blue (parental) colonies.

### Step 8: Plasmid analysis

This is done according to the ACEMBL protocols. The (optional) blue-white selection described in Step 7 should reduce the number of false positives and increase the suitability of MultiPichia for automated pipelines.

### B.1.2 Sequential assembly in MIE by SLIC

The MIE according to the present invention can also be used to integrate genes of interest (GOIs) by using multi-fragment SLIC recombination. Multigene constructs in *P. pastoris* require their own promoter-terminator cassette if are to be expressed in different translation units (for an alternative approach *vide infra).*

A detailed protocol is outlined in the following Protocol 2:

### Protocol 2. Sequential assembly by SLIC.

Reagents required:
Phusion polymerase (2 U/ul)
5X HF / GC Buffer for Phusion polymerase
dNTP mix (10 mM)
T4 DNA polymerase (3 U/ul) (and 10X buffer)
DpnI (20 U/ul)
*E. coli* competent cells
100 mM DTT, 2 M urea, 0.5 M EDTA
1 M IPTG, 100 mg/ml X-Gal or Bluo-Gal
Antibiotics

### Step 1: Primer design

The MIE element according to the present embodiment is composed of tried-and-tested primer sequences. The latter sequences comprise the "Adaptor" sequences that can be used for inserting single genes or multigene constructs. Adaptor sequences form the 5' ends of the primers used to amplify encoding DNAs to be inserted into the MIE. Insert specific sequences are appended to the 3', and other DNA sequences (e.g., Kozak sequences, purification tags) can be added optionally as desired. Since each gene insert must be included into a promoter-terminator cassette, amplification of the terminator-promoter sequences are required to stitch together the GOIs in a multigene construct (except for the first one, which uses the vector-provided promoter, and the last one, which uses the vector-provided terminator). Examples of successful adaptor sequences for a multigene construct are listed below (Table 3).

### Step 2: PCR amplification of inserts and primers

### Step 3: DpnI treatment of PCR products (optional)

### Step 4: Purification of PCR products

All the above Steps are conducted as per Protocol 1.

### Step 5: T4 DNA polymerase exonuclease treatment

### Step 6: Mixing and annealing

As in Protocol 1, Steps 5 and 6 are best done together in the "one-pot" T4 DNA exonuclease treatment, mix and anneal procedure outlined above (Protocol 1). Even though several vector-to-insert molar ratios may be tried, a 1:2 ratio (for each insert) works best for the MultiPichia vectors.

### Step 7: Transformation

### Step 8: Plasmid analysis

Transformation can be performed as per Protocol 1. As above, blue-white selection can be used to select recombinant plasmids, specifically plasmids where the LacZα fragment has been replaced by the intended multigene construct cassette. Verification of the multigene construct by sequencing and (optionally) restriction digestion is highly recommended, especially as the number of fragments to combine becomes larger.

**Table 3. Adaptor DNA sequences for single gene or multigene insertions into MultiPichia vectors by SLIC.**

| **Adaptor** | **Description** |
|---|---|
| (a) Primers for insertion of single genes into MultiPichia vectors. | |
| sAOX1InsFor (SEQ ID NO: 39) | Forward primer for insert amplification, for secretion using the α-MF sequence from *S. cerevisiae.* Needs to be followed by insert specific sequences (10-30 bp). |
| icAOX1InsFor (SEQ ID NO: 40) | Forward primer for insert amplification, for intracellular expression. The initiation ATG codon is included in the primer. Needs to be followed by insert specific sequences (10-30 bp). |
| AOX1InsRev (SEQ ID NO: 41) | Reverse primer for insert amplification (secretion or intracellular expression). Needs to be followed by insert specific sequences (10-30 bp). If a Cter-KHis10 tag is not desired, add a stop codon to the insert specific sequence (e.g., TAA). |
| AOX1VecFor (SEQ ID NO: 42) | Forward primer for vector amplification (secretion and intracellular expression), reverse complement of AOX1InsRev. |
| | No further extension required. |
| sAOX1VecRev (SEQ ID NO: 43) | Reverse primer for vector amplification for secretion expression, reverse complement of sAOX1InsFor. |
| | No further extension required. |
| icAOX1VecRev (SEQ ID NO: 44) | Reverse primer for vector amplification for intracellular expression, reverse complement of icAOX1InsFor. |
| | No further extension required. |
| (b) Primers for insertion of a multigene construct into MultiPichia vectors. Although in principle the complete AOX1 terminator-promoter adaptor fragment could be amplified as a single fragment, that strategy would result in an additional BstXI site at the terminator-promoter junction. The two-step PCR mechanism allows the reuse of multigene constructs generated as described for HE/BstXI insertion strategies (infra). | |
| ttAOX1InsVecFor (SEQ ID NO: 45) | Forward primer for amplification of an insert 1 (GOI1) / vector AOX1 terminator (secretion or intracellular) expression. Needs to be preceded by insert 1 specific sequences (10- 30 bp). |
| ttAOX1 VecRev (SEQ ID NO: 46) | Forward primer for amplification of an insert 1 (GOI1) / vector AOX1 terminator (secretion or intracellular) expression. |
| | No further extension required. |
| pAOX1VecFor (SEQ ID NO: 47) | Forward primer for vector AOX1 promoter (secretion or intracellular expression) / insert 2 (GOI2) insert. |
| | No further extension is required. |
| pAOX1VecInsRev (SEQ ID NO: 48) | Reverse primer for vector AOX1 promoter (secretion or intracellular expression) / insert 2 (GOI2) insert. Needs to be preceded by insert 2 specific sequences (10-30 bp). |

### B.1.3 Gene insertion by restriction /ligation

The MIEs of the present invention provide a minimalistic set of restriction sites that can be used for restriction / ligation cloning if so desired, yielding functional expression cassettes. Specifically, the available restriction enzyme sites are Ncol and KpnI (5', secretion), BstBI (5', intracellular), and Pmel and MscI (3'). As with the seamless cloning strategies, in constructs targeted for secretion expression a choice is possible between Nter-His6-3C tags by using a 5' KpnI site in the PCR fragment and a Cter-KHis10 tag using a 3' Pmel site and no stop codon in the PCR fragment. Untagged N-terminal or C-terminal fragments are generated when Ncol is used for the 5' end of the PCR fragment and MscI / Pmel plus a stop codon is used for the 3' end of the PCR fragment. In constructs for intracellular expression the only available 5' site is BstBI, which yields N-terminally untagged protein by default.

Genes of interest (GOI) can be subcloned into the MIEs by using standard cloning strategies of the MultiPichia vectors.

### Protocol 3. Restriction / ligation cloning into the MIE.

Reagents required:
Phusion polymerase (2 U/ul)
5X HF or GC Buffer for Phusion polymerase
dNTP mix (10 mM)
10 mM BSA
Ncol, KpnI, BstBI, Pmel, and/or MscI (and 10X Buffer)
T4 DNA ligase (and 10X buffer)
Antarctic Phosphatase (and 5X Buffer)
*E. coli* competent cells
1 M IPTG, 100 mg/ml X-Gal or Bluo-Gal
Antibiotics

### Step 1: Primer design

For conventional cloning, PCR primers are designed such that they contain appropriate restriction sites, preceded by suitably chosen overhangs for efficient cleavage (e.g., NEB catalogue), and followed by ≥20 nucleotides overlapping with the gene of interest to be inserted.

All MultiPichia Donor and Acceptor vectors share the same restriction sites and MIEs; therefore they support classical cloning in a uniform manner for single-gene insertions. No additional sequences are required for secretion / intracellular expression constructs.

### Step 2: Insert preparation

### Step 3: Vector preparation

### Step 4: Ligation

Steps 2-4 can be performed with the MultiPichia vectors as for the ACEMBL vectors, without modifications.

### Step 5: Transformation

### Step 6: Plasmid analysis

Steps 5 and 6 can be performed with the MultiPichia vectors as for the ACEMBL vectors. Blue-white selection is possible, as with the seamless cloning approach.

### B.1.4 Multiplication by using the HE and BstXI sites

The MultiPichia Donor and Acceptor vectors contain MIE comprising a homing endonuclease (HE) site 5' to the promoter sequence and a especially chosen BstXI site 3' to the terminator sequence. I-Scel and a compatible BstXI site are in the Acceptor vectors and PI-SceI and a compatible BstXI site are in the Donor vectors. Each HE/BstXI site will generate compatible overhangs upon cleavage. When a HE/BstXI digested fragment is ligated into a HE site of a MultiPichia vector the asymmetry of the recognition sites ensures that the ligated extremes cannot be cut by any of the enzymes (the ligated HE and BstXI sites are "destroyed"). In this manner, successive rounds of multiplication can be performed iteratively.

### Protocol 4. Multiplication by using HE / BstXI.

Reagents required:
Homing endonucleases I-SceI, PI-SceI (and 10X Buffer)
Restriction enzyme BstXI (and 10X Buffer)
T4 DNA ligase (and 10X buffer)
Antarctic Phosphatase (and 5X Buffer)
*E. coli* competent cells
Antibiotics

### Step 1: Primer design

This Step is no longer necessary since all manipulations involve restriction digests and ligations.

### Step 2: Insert preparation

### Step 3: Vector preparation

### Step 4: Ligation

### Step 5: Transformation

### Step 6: Plasmid analysis

### B.2 Cre-LoxP reaction of Acceptors and Donors

All the MultiPichia Acceptor and Donor vectors contain the LoxP imperfect inverted repeat sequence (SEQ ID NO: 49). Cre-IoxP recombination to obtain fusion vectors may be performed as described in WO2010100278. In brief, recombination of two or more vectors bearing the LoxP sequence catalyzed by the Cre recombinase results in an equilibrium between all possible fusion vectors. Specific fusion vectors can be rescued by transforming the mixture in *E. coli cells* and plating them on appropriate antibiotics.

In contrast to WO2010100278, which discloses vectors for bacterial expression, the MultiPichia Acceptor vectors are the only ones that carry selection markers for yeast; therefore plasmid fusions must contain at least one Acceptor vector.

Fusion vectors in excess of 30 kb can be thus generated. The stability of these multigene constructs once inserted in *P. pastoris* genome can be tested by PCR amplification after serial passaging for more than 10 generations. Such stability tests show that the MultiPichia genome inserts are stable even when the selection antibiotic (e.g., Zeocin) is not present in the growth medium.

Since MultiPichia plasmids should be linearized for insertion into *P. pastoris* genome preferably by using SacI, very large fusion plasmids should be constructed that do not have SacI cleavage sites. This is most easily accomplished by custom gene synthesis and/or site-directed mutagenesis to remove any SacI sites present in the genes of interest.

### C. MultiPichia kit for expression of proteins in P. pastoris

A kit according to the preferred embodiment for expression in *P. pastoris* contains:
- BW23473, BW23474 cells¹ and/or Cre recombinase.
- pPACE, pPDK, pPDC, pPDS vectors
- pBiPACE vector
   ¹ *E. coli* strains expressing the pir gene for propagation of Donor derivatives (any *pir*+ *E. coli* strain could be used).

### Optional components:

- Antibiotics: zeocin, chloramphenicol, kanamycin, spectinomycin.
- Enzymes:
   T4 DNA polymerase (for recombination insertion of genes) Phusion polymerase (for PCR amplification of DNA) Restriction enzymes and T4 DNA ligase (for conventional cloning, if desired)

Throughout the description and claims the word "comprise" and variations of the word, are not intended to exclude other technical features, additives, components, or steps. Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### EXAMPLES

Examples of multiprotein expressions by using the above-described MultiPichia system are shown to illustrate the gene combination procedures outlined above. Reactions presented were carried out manually following the protocols provided in the above Section B.

### Example 1: SLIC cloning into MultiPichia vectors: HPV16L1 and HPV18L1

Genes coding for full-length herpes virus-like particles (VLPs) 16L1 and 18L1 proteins (HPV16L1 and HPV18L1, respectively) were amplified from custom synthesized DNAs (Genscript) by using the following primer pairs for HPV16L1: icAOX1 InsForHPV16L1 (SEQ IS NO: 51), which is a derivative of the icAOX1InsFor adaptor sequence (Table 3) elongated at its 3' end with the 5' end of the HPV16L1 gene, and AOX1InsRevHPV16L1 (SEQ ID NO: 52), a derivative of the AOX1InsRev adaptor sequence (Table 3) elongated at its 3' end with the 3' end of the HPV16L1 gene; and likewise for HPV18L1: icAOX1InsForHPV18L1 (SEQ ID NO: 53) and AOX1 InsRevHPV18L1 (SEQ ID NO: 54), which are derivatives of the corresponding adaptors (Table 3). Both synthetic genes were amplified and prepared following the protocols described above. Linearized vector backbones were generated by digestion of pPACE with BstBI-PmeI. Above Protocol 1 (Section B.1.1) was followed, resulting in pPACE-HPV16L1his and pPACE-HPV18L1his (Figure 5; "his" means Cter-KHis10 tagged). The same constructs but with a stop codon were also prepared using above Protocol 1 (Section B.1.1), resulting in pPACE-HPV16L1 and pPACE-HPV18L1 (Figure 5). One clone per construct was linearized with SacI and electroporated into *P. pastoris* SMD1168 cells. Yeast colonies were analyzed to find positive transformants. One transformant per construct was used for expression of tagged and untagged HPV16L1 and HPV18L1 proteins, which were purified by cation-exchange capture (5-ml HiTrap S XL, GE Healthcare) and S200 (Superdex 200, GE Healthcare) chromatography, resulting in pure proteins (Figure 5). All four proteins eluted from the S200 column with the expected molecular size (pentameric). For HPV16L1 and HPV18L1, the untagged versions were more stable than the corresponding oligohistidine tagged versions, which shows some proteolytic degradation.

The flexibility and convenience of the design of the MultiPichia Acceptor vector is illustrated by the facile choice of untagged or C-terminally tagged fusion constructs by including (or not) a stop codon at the cloning stage; for these targets, this minimal difference proved critical for the stability of the expressed product.

### Example 2: SLIC cloning into pBiPACE and transient expression: Sch. pombe PSAT

The gene coding for full-length Schizosaccharomyces pombe phosphoserine aminotransferase (PSAT, SerC) (amino acids 1-389) was amplified from pOPINF-PS using icAOX1InsForPS (SEQ ID NO: 55) and AOX1InsRevPS (SEQ ID NO: 56), which are derivative of the adaptor sequences (Table 3) elongated at their 3' ends with 5' and 3' insert specific sequences. The forward PCR primer is partially complementary to the N-terminal His6-3C tag encoded by pOPINF-PS, hence the resultant PCR amplicon contains an affinity oligohistidine tag; to avoid a second oligohistidine tag in the construct, the reverse primer insert a stop codon (TAA) at the 3' end of the coding sequence. The PSAT gene (SEQ ID NO: 57) was amplified following the protocols above and inserted into the pBiPACE Acceptor vector previously linearized with BstBI-PmeI, following above Protocol 1 (Section B.1.1), resulting in pBiPACE-hisPS (Figure 6). The construct's identity was verified by sequencing. The pBiPACE-hisPS plasmid was electroporated in *P*. *pastoris* SMD1168 cells, where it is maintained episomally. One colony from a zeocin-containing plate was used for expression of hisPS, which was purified by Ni²⁺ Immobilized metal ion affinity chromatography (IMAC) capture and S200 size exclusion chromatography, resulting in pure protein (Figure 6).

### Example 3: Construction of a vector containing multiple expression cassettes for co-expression of anti-MUC1 IIB6 scFv and PpHAC1

Plasmids pPACE-αMUC1his (encoding a Cter-KHis8 tagged scFv derived from the αMUC1 IIB6 antibody, SEQ ID NO: 58) and pPACEΔSacI-HAC1 (encoding the *P. pastoris* HAC1 transcription factor, SEQ ID NO: 59 cloned in a pPACE vector where the SacI site in the AOX1 promoter has been mutated) were prepared following the methods described above (Section B.1). The gene expression cassette encoding for *Pp*HAC1 (comprising AOX1ΔSacI promoter, *Pp*HAC1 gene, and the AOX1 terminator) was excised by digestion with I-CeuI and BstXI. The liberated fragment was inserted into I-CeuI digested and Antarctic-phosphatase treated pPACE-αMUC1his resulting in pPACE-αMUC1his-HAC1. The ligation mixture was then transformed into *E. coli* cells, the combined plasmid was recovered by plating on zeocin plates and verified by restriction digestion. The combined plasmid was linearized with SacI and electroporated into *P*. *pastoris* SMD1168 cells. Expression and purification by Ni²⁺ capture (IMAC) and S200 size exclusion chromatography resulted in enhanced secretion of anti-MUC1 IIB6 scFv (Figure 7).

### Example 4: Construction of a vector containing multiple expression cassettes by Acceptor-donor fusion and co-expression of anti-MUC1 IIB6 scFv and PpHAC1

Plasmids pPACE-αMUC1his (encoding a Cter-KHis8 tagged scFv derived from the αMUC1 IIB6 antibody, SEQ ID NO: 58, targeted for secretion with the αMF leader sequence) and pPDCΔSacI-HAC1 (encoding the *P. pastoris* HAC1 transcription factor, SEQ ID NO: 59, cloned in a pPDC vector where the SacI site in the AOX1 promoter has been mutated), prepared following the methods described above (Section B.1), were assembled as illustrated in Figure 8. Following above methods (Section B.2), Cre recombination was used to generate the fusion plasmid from the two individual expression constructs, which was selected on LB agar plates with zeocin and chloramphenicol. Clones were verified by restriction analysis. The combined plasmid was linearized with SacI and electroporated into *P. pastoris* SMD1168cells. Expression and Ni²⁺ IMAC capture and S200 size exclusion chromatography resulted in highly levels of secreted anti-MUC1 IIB6 scFv (Figure 8).

### Example 5: Coexpression from Acceptor-Donor fusions: human fB / Furin

Plasmids pPACE-fB (encoding a Nter-His6 tagged human complement factor B, fB, SEQ ID NO: 60, targeted for secretion with the αMF leader sequence) and pPDCΔSacI-flagFurin (encoding Nter-FLAG Furin, SEQ ID NO: 61, cloned in a pPDC vector where the SacI site in the AOX1 promoter has been mutated), prepared following the methods described above (Section B.1), were assembled as illustrated in Figure 9. Following above methods (Section B.2), Cre recombination was used to generate the fusion plasmid from the two individual expression constructs, which was selected on LB agar plates with zeocin and chloramphenicol. Clones were verified by restriction analysis. The combined plasmid was linearized with SacI and electroporated into *P*. *pastoris* SMD1168 cells. Expression and Anti-FLAG affinity capture and S200 size exclusion chromatography resulted in highly levels of secreted human fB (Figure 9). Functional assays verified that the recombinant protein has authentic activity towards its cognate substrate (C3) (Figure 9).

### Example 6: Pichia pastoris genome editing with CRISPR/Cas9

The modular design of the MultiPichia plasmids allow the generation of recombinant plasmids for applications other than recombinant protein production, such as genome editing and cell strain engineering. Plasmid pBiPACE-Cas9GFP-gRNA (Figure 10) was prepared following the methods above (Section B.1), where the Cas9GFP-gRNA insert, SEQ ID NO: 62, was synthesized as a single DNA molecule and inserted by restriction-ligation into the MIE between the pBiPACE vector's BstBI and Pmel sites. The guide RNA (gRNA) encoded in the insert served to direct the Cas9GFP protein to a specific genomic locus where a mutation was introduced. The plasmid was electroporated into *P. pastoris* SMD1168 cells and induction of Cas9 was trigged by methanol induction. The successful introduction of the sequence substitution encoded in the gRNA DNA fragment was confirmed by sequencing, thereby demonstrating the possibility of performing genome engineering in *P. pastoris* with the CRISPR/Cas9 system.

### REFERENCES CITED IN THE APPLICATION

Fitzgerald et al, "Protein complex expression by using multigene baculoviral vectors". Nat. Methods, 2006, vol. 3, p. 1021 -1032
Berger et al, "Automated unrestricted multigene recombineering for multiprotein complex production", Nat Methods, 2009, vol. 6, p. 447-450
WO2010100278
Bieniossek, C., et al. "Automated unrestricted multigene recombineering for multiprotein complex production", Nat Methods 2009, vol. 6, p. 447-450.
Michael R. Green & Joseph Sambrook (2012). Molecular Cloning: a Laboratory Manual, 4th edn. Cold Spring Harbor, NY: Cold Spring Harbor Laboratory
Li, M.Z., and Elledge, S.J. "Harnessing homologous recombination in vitro to generate recombinant DNA via SLIC", Nat Methods 2007, vol. 4, p. 251-256.

## Claims

1. An isolated multiple integration element (MIE) comprising, in the specified order:
a) a homing endonuclease site (HE);
b) a promoter sequence selected from the group consisting of *AOX1, FLD1, FMD1, GAP* and *TEF1,* wherein the promoter sequence is devoid of HE, BstBI, Ncol, Pmel and BstXI restriction sites if said restriction sites are present;
c) a BstBI restriction site;
d) a sequence encoding a secretion signal;
e) a Ncol restriction site;
f) a selection marker gene;
g) a Pmel restriction site;
h) a sequence encoding a detection and/or purification polypeptide;
i) a terminator sequence selected from the group consisting of *AOX1, FLD1, FMD1, GAP* and *TEF1,* wherein the terminator sequence is devoid of HE, BstBI, Ncol, Pmel and BstXI restriction sites if said restriction sites are present; and
j) a BstXI restriction site,
wherein the HE and BstXI sites are selected such that HE and BstXI result in compatible cohesive ends when cut by the homing endonuclease and the BstXI restriction enzyme, respectively, and the ligation product of HE and BstXI cohesive ends can neither be cleaved by the homing endonuclease nor the BstXI restriction enzyme.

2. The isolated MIE according to claim 1, wherein the promoter and terminator are AOX1 promoter and AOX1 terminator or, alternatively, wherein the secretion signal sequence is *S. cerevisiae* alpha-mating factor or, alternatively, wherein the promoter is AOX1 promoter, the terminator is AOX1 terminator and the secretion signal sequence is *S. cerevisiae* alpha-mating factor.

3. The isolated MIE according to any of the claims 1-2, wherein the selection marker gene is LacZ or, alternatively, wherein the detection or purification polypeptide is selected from the group consisting of poly-histidine (6-20 histidine residues), FLAG-tag, StrepII-tag, HA epitope, c-Myc epitope, V5 epitope, calmodulin-binding peptide (CBP), enhanced green fluorescent protein (EGFP), enhanced yellow fluorescent protein (EYGP), Rudolph red fluorescent protein (RFP), monomeric Cherry fluorescent protein (mCherry), and cyan fluorescent protein (CFP) or, alternatively, wherein the selection marker gene is LacZ and the detection or purification polypeptide is selected from the group consisting of poly-histidine (6-20 histidine residues), FLAG-tag, StrepII-tag, HA epitope, c-Myc epitope, V5 epitope, calmodulin-binding peptide (CBP), enhanced green fluorescent protein (EGFP), enhanced yellow fluorescent protein (EYGP), Rudolph red fluorescent protein (RFP), monomeric Cherry fluorescent protein (mCherry), and cyan fluorescent protein (CFP).

4. The isolated MIE according to any of the claims 1-3, wherein the HE site is selected from the group consisting of I-CeuI site, a PI-SceI site, a I-SceI site, a I-PpoI site, a I-CreI site, and a I-DmoI site.

5. The isolated MIE according to any of the claims 1-4, wherein the HE/BstXI restriction sites are selected from the following HE/BstXI pairs: SEQ ID NO: 3/SEQ ID NO: 4, SEQ ID NO: 7/SEQ ID NO: 8, SEQ ID NO: 11/SEQ ID NO: 12, SEQ ID NO: 15/SEQ ID NO: 16, SEQ ID NO: 19/SEQ ID NO: 20, SEQ ID NO: 23/SEQ ID NO: 24, SEQ ID NO: 27/SEQ ID NO: 28, or their complementary sequences.

6. The isolated MIE according to any of the claims 1-5, which is one consisting of sequence SEQ ID NO: 31 or SEQ ID NO: 32.

7. An expression cassette derived from the MIE as defined in any of the claims 1-6 comprising one or more genes of interest.

8. A vector comprising the MIE according to any of the claims 1-6 or the expression cassette according to claim 7.

9. The vector according to claim 8, further comprising at least one of the sequence elements consisting of an antibiotic resistance marker, an origin of replication and from 1 to 6 sites for integration of the vector into another vector or host cell.

10. A vector according to claim 9, wherein the site for integration is loxP.

11. The vector according to any of the claims 7-10, further comprising a PARS1 sequence.

12. The vector according to any of the claims 7-11 consisting of SEQ ID NO: 34, SEQ ID NO: 35, SEQ ID NO: 36, SEQ ID NO: 37 or SEQ ID NO: 38.

13. A host cell containing the MIE according to any of the claims 1-5, the expression cassette according to claim 6, or the vector according to any of the claims 7-12.

14. A kit for cloning and/or expressing proteins containing at least one vector according to any of the claims 9-12.

15. A method for producing multiple protein complexes comprising the steps of:
(i) producing a vector comprising from 1 to 10 expression cassettes as defined in claim 7
(ii) introducing the vector obtained in step (i) into a *P. pastoris* host cell; and
(iii) incubating the *P. pastoris* host cell under conditions allowing the simultaneous expression of the genes present in the vector.


SEQUENCE LISTING

<110>  Consejo Superior de Investigaciones Científicas
       European Molecular Biology Laboratory

<120>  Tools for multiprotein complex expression in Pichia pastoris

<130>  P3218EP00

<160>  62

<170>  PatentIn version 3.5

<210>  1
<211>  944
<212>  DNA
<213>  Artificial Sequence

<220>
<223>  Engineered AOX1 promoter

<400>  1
ctagagatct aacatccaaa gacgaaaggt tgaatgaaac ctttttgcca tccgacatcc      60

acaggtccat tctcacacat aagtgccaaa cgcaacagga ggggatacac tagcagcaga     120

ccgttgcaaa cgcaggacct ccactcctct tctcctcaac acccactttt gccatcgaaa     180

aaccagccca gttattgggc ttgattggag ctcgctcatt ccaattcctt ctattaggct     240

actaacacca tgactttatt agcctgtcta tcctggcccc cctggcgagg ttcatgtttg     300

tttatttccg aatgcaacaa gctccgcatt acacccgaac atcactccag atgagggctt     360

tctgagtgtg gggtcaaata gtttcatgtt ccccaaatgg cccaaaactg acagttataa     420

cgctgtcttg gaacctaata tgacaaaagc gtgatctcat ccaagatgaa ctaagtttgg     480

ttcgttgaaa tgctaacggc cagttggtca aaaagaaact tccaaaagtc ggcataccgt     540

ttgtcttgtt tggtattgat tgacgaatgc tcaaaaataa tctcattaat gcttagcgca     600

gtctctctat cgcttctgaa ccccggtgca cctgtgccga aacgcaaatg gggaaacacc     660

cgctttttgg atgattatgc attgtctcca cattgtatgc ttcctagatt ctggtgggaa     720

tactgctgat agcctaacgt tcatgatcaa aatttaactg ttctaacccc tacttgacag     780

caatatataa acagaaggaa gctgccctgt cttaaacctt tttttttatc atcattatta     840

gcttactttc ataattgcga ctggttccaa ttgacaagct tttgatttta acgactttta     900

acgacaactt gagaagatca aaaaacaact aattattcga aacg                      944


<210>  2
<211>  12
<212>  DNA
<213>  Artificial Sequence

<220>
<223>  BstXI recognition sequence

<220>
<221>  misc_feature
<222>  (4)..(9)
<223>  n is a, c, g, or t

<400>  2
ccannnnnnt gg                                                          12


<210>  3
<211>  27
<212>  DNA
<213>  Artificial Sequence

<220>
<223>  I-CeuI restriction site

<400>  3
taactataac ggtcctaagg tagcgac                                          27


<210>  4
<211>  12
<212>  DNA
<213>  Artificial Sequence

<220>
<223>  BStXI restriction site

<400>  4
ccatctaatt gg                                                          12


<210>  5
<211>  27
<212>  DNA
<213>  Artificial Sequence

<220>
<223>  I-CeuI restriction site

<400>  5
cgctacctta ggaccgttat agttatg                                          27


<210>  6
<211>  12
<212>  DNA
<213>  Artificial Sequence

<220>
<223>  BstXI restriction site

<400>  6
ggtagattaa cc                                                          12


<210>  7
<211>  37
<212>  DNA
<213>  Artificial Sequence

<220>
<223>  PI-SceI restriction site

<400>  7
atctatgtcg ggtgcggaga aagaggtaat gaaatgg                                37


<210>  8
<211>  12
<212>  DNA
<213>  Artificial Sequence

<220>
<223>  BstXI restriction site

<400>  8
ccatgtgcct gg                                                           12


<210>  9
<211>  37
<212>  DNA
<213>  Artificial Sequence

<220>
<223>  PI-SceI restriction site

<400>  9
tagatacagc ccacgcctct ttctccatta ctttacc                                37


<210>  10
<211>  12
<212>  DNA
<213>  Artificial Sequence

<220>
<223>  BstXI restriction site

<400>  10
ggtacacgga cc                                                           12


<210>  11
<211>  30
<212>  DNA
<213>  Artificial Sequence

<220>
<223>  PI-PspI restriction site

<400>  11
tggcaaacag ctattatggg tattatgggt                                        30


<210>  12
<211>  12
<212>  DNA
<213>  Artificial Sequence

<220>
<223>  BstXI restriction site

<400>  12
ccatttatct gg                                                          12


<210>  13
<211>  30
<212>  DNA
<213>  Artificial Sequence

<220>
<223>  PI-PspI restriction site

<400>  13
accgtttgtc gataataccc ataataccca                                       30


<210>  14
<211>  12
<212>  DNA
<213>  Artificial Sequence

<220>
<223>  BstXI restriction site

<400>  14
ggtaaataga cc                                                          12


<210>  15
<211>  18
<212>  DNA
<213>  Artificial Sequence

<220>
<223>  I-SceI restriction site

<400>  15
tagggataac agggtaat                                                    18


<210>  16
<211>  12
<212>  DNA
<213>  Artificial Sequence

<220>
<223>  BstXI restriction site

<400>  16
ccatataact gg                                                          12


<210>  17
<211>  18
<212>  DNA
<213>  Artificial Sequence

<220>
<223>  I-SceI restriction site

<400>  17
atccctattg tcccatta                                                    18

<210>  18
<211>  12
<212>  DNA
<213>  Artificial Sequence

<220>
<223>  BstXI restriction site

<400>  18
ggtatattga cc                                                          12


<210>  19
<211>  15
<212>  DNA
<213>  Artificial Sequence

<220>
<223>  I-PpoI restriction site

<400>  19
ctctcttaag gtagc                                                       15


<210>  20
<211>  12
<212>  DNA
<213>  Artificial Sequence

<220>
<223>  BstXI restriction site

<400>  20
ccatttaact gg                                                          12


<210>  21
<211>  15
<212>  DNA
<213>  Artificial Sequence

<220>
<223>  I-PpoI restriction site

<400>  21
gagagaattc catcg                                                       15


<210>  22
<211>  12
<212>  DNA
<213>  Artificial Sequence

<220>
<223>  BstXI restriction site

<400>  22
ggtaaattga cc                                                          12


<210>  23
<211>  30

<212>  DNA
<213>  Artificial Sequence

<220>
<223>  I-CreI restriction site

<400>  23
ctgggttcaa aacgtcgtga gacagtttgg                                        30


<210>  24
<211>  12
<212>  DNA
<213>  Artificial Sequence

<220>
<223>  BstXI restriction site

<400>  24
ccatgtgact gg                                                           12


<210>  25
<211>  30
<212>  DNA
<213>  Artificial Sequence

<220>
<223>  I-CreI restriction site

<400>  25
gacccaagtt ttgcagcact ctgtcaaacc                                        30


<210>  26
<211>  12
<212>  DNA
<213>  Artificial Sequence

<220>
<223>  BstXI restriction site

<400>  26
ggtacactga cc                                                           12


<210>  27
<211>  30
<212>  DNA
<213>  Artificial Sequence

<220>
<223>  I-DmoI restriction site

<400>  27
atgccttgcc gggtaagttc cggcgcgcat                                        30


<210>  28
<211>  12
<212>  DNA
<213>  Artificial Sequence

<220>
<223>  BstXI restriction site

<400>  28
ccatgtaact gg                                                          12


<210>  29
<211>  30
<212>  DNA
<213>  Artificial Sequence

<220>
<223>  I-DmoI restriction site

<400>  29
tacggaacgg cccattcaag gccgcgcgta                                       30


<210>  30
<211>  12
<212>  DNA
<213>  Artificial Sequence

<220>
<223>  BstXI restriction site

<400>  30
ggtacattga cc                                                          12


<210>  31
<211>  2034
<212>  DNA
<213>  Artificial Sequence

<220>
<223>  Multiple Integration Element (I-CeuI is HE site)

<400>  31
atgcataact ataacggtcc taaggtagcg acctagagat ctaacatcca aagacgaaag      60

gttgaatgaa acctttttgc catccgacat ccacaggtcc attctcacac ataagtgcca     120

aacgcaacag gaggggatac actagcagca gaccgttgca aacgcaggac ctccactcct     180

cttctcctca acacccactt ttgccatcga aaaaccagcc cagttattgg gcttgattgg     240

agctcgctca ttccaattcc ttctattagg ctactaacac catgacttta ttagcctgtc     300

tatcctggcc cccctggcga ggttcatgtt tgtttatttc cgaatgcaac aagctccgca     360

ttacacccga acatcactcc agatgagggc tttctgagtg tggggtcaaa tagtttcatg     420

ttccccaaat ggcccaaaac tgacagttat aacgctgtct tggaacctaa tatgacaaaa     480

gcgtgatctc atccaagatg aactaagttt ggttcgttga aatgctaacg gccagttggt     540

caaaaagaaa cttccaaaag tcggcatacc gtttgtcttg tttggtattg attgacgaat     600

gctcaaaaat aatctcatta atgcttagcg cagtctctct atcgcttctg aaccccggtg     660

cacctgtgcc gaaacgcaaa tggggaaaca cccgcttttt ggatgattat gcattgtctc     720

cacattgtat gcttcctaga ttctggtggg aatactgctg atagcctaac gttcatgatc 780

aaaatttaac tgttctaacc cctacttgac agcaatatat aaacagaagg aagctgccct 840

gtcttaaacc ttttttttta tcatcattat tagcttactt tcataattgc gactggttcc 900

aattgacaag cttttgattt taacgacttt taacgacaac ttgagaagat caaaaaacaa 960

ctaattattc gaaacgatga gatttccatc tattttcaca gctgttttgt ttgctgcttc 1020

ttccgctttg gctgctccag ttaacactac aactgaagat gagactgctc aaattcctgc 1080

tgaagctgtt atcggttact tggacttgga gggagatttt gacgttgctg ttttgccttt 1140

ctcaaactct acaaacaacg gtttgttgtt catcaacaca actatcgctt ccatcgctgc 1200

taaggaagag ggagtttcat tggataaaag agaagctgag gccatggcac accatcacca 1260

ccatcacagc agcggtaccg tcgacccgac tggaaagcgg gcagtgagcg caacgcaatt 1320

aatgtgagtt agctcactca ttaggcaccc caggctttac actttatgct tccggctcgt 1380

atgttgtgtg gaattgtgag cggataacaa tttcacacag gaaacagcta tgaccatgat 1440

tacggattca ctggccgtcg ttttacaacg tcgtgactgg gaaaaccctg gcgttaccca 1500

acttaatcgc cttgcagcac atcccccttt cgccagctgg cgtaatagcg aagaggcccg 1560

caccgatcgc ccttcccaac agttgcgcag cctgaatggc gaatggcgcc aagctttcta 1620

gaccagttta aacatcacca tcaccatcat catcaccatc actaatggcc atcaagagga 1680

tgtcagaatg ccatttgcct gagagatgca ggcttcattt ttgatacttt tttatttgta 1740

acctatatag tataggattt tttttgtcat tttgtttctt ctcgtacgag cttgctcctg 1800

atcagcctat ctcgcagctg atgaatatct tgtggtaggg gtttgggaaa atcattcgag 1860

tttgatgttt ttcttggtat ttcccactcc tcttcagagt acagaagatt aagtgacacg 1920

ttcgtttgtg caagcttcaa cgatgccaaa agggtataat aagcgtcatt tgcagcattg 1980

tgaagaaaac tatgtggcaa gccaagcctg cgaagaatgt accatctaat tggg 2034

<210> 32
<211> 2038
<212> DNA
<213> Artificial Sequence

<220>
<223> Multiple Integration Element (PI-SceI is HE; SacI deleted)

<400> 32
atctatgtcg ggtgcggaga aagaggtaat gaaatggcta gagatctaac atccaaagac 60

gaaaggttga atgaaacctt tttgccatcc gacatccaca ggtccattct cacacataag 120

tgccaaacgc aacaggaggg gatacactag cagcagaccg ttgcaaacgc aggacctcca 180

ctcctcttct cctcaacacc cacttttgcc atcgaaaaac cagcccagtt attgggcttg 240

attggagcta gctcattcca attccttcta ttaggctact aacaccatga ctttattagc 300

ctgtctatcc tggcccccct ggcgaggttc atgtttgttt atttccgaat gcaacaagct 360

ccgcattaca cccgaacatc actccagatg agggctttct gagtgtgggg tcaaatagtt 420

tcatgttccc caaatggccc aaaactgaca gttataacgc tgtcttggaa cctaatatga 480

caaaagcgtg atctcatcca agatgaacta agtttggttc gttgaaatgc taacggccag 540

ttggtcaaaa agaaacttcc aaaagtcggc ataccgtttg tcttgtttgg tattgattga 600

cgaatgctca aaaataatct cattaatgct tagcgcagtc tctctatcgc ttctgaaccc 660

cggtgcacct gtgccgaaac gcaaatgggg aaacacccgc tttttggatg attatgcatt 720

gtctccacat tgtatgcttc ctagattctg gtgggaatac tgctgatagc ctaacgttca 780

tgatcaaaat ttaactgttc taacccctac ttgacagcaa tatataaaca gaaggaagct 840

gccctgtctt aaaccttttt ttttatcatc attattagct tactttcata attgcgactg 900

gttccaattg acaagctttt gattttaacg acttttaacg acaacttgag aagatcaaaa 960

aacaactaat tattcgaaac gatgagattt ccatctattt tcacagctgt tttgtttgct 1020

gcttcttccg ctttggctgc tccagttaac actacaactg aagatgagac tgctcaaatt 1080

cctgctgaag ctgttatcgg ttacttggac ttggagggag attttgacgt tgctgttttg 1140

cctttctcaa actctacaaa caacggtttg ttgttcatca acacaactat cgcttccatc 1200

gctgctaagg aagagggagt ttcattggat aaaagagaag ctgaggccat ggcacaccat 1260

caccaccatc acagcagcgg taccgtcgac ccgactggaa agcgggcagt gagcgcaacg 1320

caattaatgt gagttagctc actcattagg caccccaggc tttacacttt atgcttccgg 1380

ctcgtatgtt gtgtggaatt gtgagcggat aacaatttca cacaggaaac agctatgacc 1440

atgattacgg attcactggc cgtcgtttta caacgtcgtg actgggaaaa ccctggcgtt 1500

acccaactta atcgccttgc agcacatccc cctttcgcca gctggcgtaa tagcgaagag 1560

gcccgcaccg atcgcccttc ccaacagttg cgcagcctga atggcgaatg gcgccaagct 1620

ttctagacca gtttaaacat caccatcacc atcatcatca ccatcactaa tggccatcaa 1680

gaggatgtca gaatgccatt tgcctgagag atgcaggctt catttttgat acttttttat 1740

ttgtaaccta tatagtatag gatttttttt gtcattttgt ttcttctcgt acgagcttgc 1800

tcctgatcag cctatctcgc agctgatgaa tatcttgtgg taggggtttg ggaaaatcat 1860

tcgagtttga tgtttttctt ggtatttccc actcctcttc agagtacaga agattaagtg 1920

acacgttcgt ttgtgcaagc ttcaacgatg ccaaaagggt ataataagcg tcatttgcag 1980

cattgtgaag aaaactatgt ggcaagccaa gcctgcgaag aatgtaccat gtgcctgg 2038

<210> 33
<211> 375

<212>  DNA
<213>  Artificial Sequence

<220>
<223>  Engineered Zeocin resistance gene

<400>  33
atggctaagt tgacttccgc tgttccagtt ttgacagcta gagatgttgc tggtgctgtt      60

gaattttgga ctgatagatt gggattttca agagacttcg ttgaggatga ctttgctggt     120

gttgttagag atgacgttac tttgttcatt tctgctgttc aagatcaggt tgttcctgac     180

aacacattgg cttgggtttg ggttagaggt ttggatgaat tgtacgctga atggtctgag     240

gttgtttcca ctaatttcag agacgcttct ggaccagcta tgacagaaat cggagagcaa     300

ccttggggaa gagagtttgc tttgagagat ccagctggaa actgtgttca tttcgttgct     360

gaagagcagg actaa                                                      375


<210>  34
<211>  4032
<212>  DNA
<213>  Artificial Sequence

<220>
<223>  pPACE

<400>  34
atgcataact ataacggtcc taaggtagcg acctagagat ctaacatcca aagacgaaag      60

gttgaatgaa acctttttgc catccgacat ccacaggtcc attctcacac ataagtgcca     120

aacgcaacag gaggggatac actagcagca gaccgttgca aacgcaggac ctccactcct     180

cttctcctca acacccactt ttgccatcga aaaaccagcc cagttattgg gcttgattgg     240

agctcgctca ttccaattcc ttctattagg ctactaacac catgacttta ttagcctgtc     300

tatcctggcc cccctggcga ggttcatgtt tgtttatttc cgaatgcaac aagctccgca     360

ttacacccga acatcactcc agatgagggc tttctgagtg tggggtcaaa tagtttcatg     420

ttccccaaat ggcccaaaac tgacagttat aacgctgtct tggaacctaa tatgacaaaa     480

gcgtgatctc atccaagatg aactaagttt ggttcgttga aatgctaacg gccagttggt     540

caaaaagaaa cttccaaaag tcggcatacc gtttgtcttg tttggtattg attgacgaat     600

gctcaaaaat aatctcatta atgcttagcg cagtctctct atcgcttctg aaccccggtg     660

cacctgtgcc gaaacgcaaa tggggaaaca cccgcttttt ggatgattat gcattgtctc     720

cacattgtat gcttcctaga ttctggtggg aatactgctg atagcctaac gttcatgatc     780

aaaatttaac tgttctaacc cctacttgac agcaatatat aaacagaagg aagctgccct     840

gtcttaaacc ttttttttta tcatcattat tagcttactt tcataattgc gactggttcc     900

aattgacaag cttttgattt taacgacttt taacgacaac ttgagaagat caaaaaacaa     960

ctaattattc gaaacgatga gatttccatc tattttcaca gctgttttgt ttgctgcttc 1020

ttccgctttg gctgctccag ttaacactac aactgaagat gagactgctc aaattcctgc 1080

tgaagctgtt atcggttact tggacttgga gggagatttt gacgttgctg ttttgccttt 1140

ctcaaactct acaaacaacg gtttgttgtt catcaacaca actatcgctt ccatcgctgc 1200

taaggaagag ggagtttcat tggataaaag agaagctgag gccatggcac accatcacca 1260

ccatcacagc agcggtaccg tcgacccgac tggaaagcgg gcagtgagcg caacgcaatt 1320

aatgtgagtt agctcactca ttaggcaccc caggctttac actttatgct tccggctcgt 1380

atgttgtgtg gaattgtgag cggataacaa tttcacacag gaaacagcta tgaccatgat 1440

tacggattca ctggccgtcg ttttacaacg tcgtgactgg gaaaaccctg gcgttaccca 1500

acttaatcgc cttgcagcac atcccccttt cgccagctgg cgtaatagcg aagaggcccg 1560

caccgatcgc ccttcccaac agttgcgcag cctgaatggc gaatggcgcc aagctttcta 1620

gaccagttta aacatcacca tcaccatcat catcaccatc actaatggcc atcaagagga 1680

tgtcagaatg ccatttgcct gagagatgca ggcttcattt ttgatacttt tttatttgta 1740

acctatatag tataggattt tttttgtcat tttgtttctt ctcgtacgag cttgctcctg 1800

atcagcctat ctcgcagctg atgaatatct tgtggtaggg gtttgggaaa atcattcgag 1860

tttgatgttt ttcttggtat ttcccactcc tcttcagagt acagaagatt aagtgacacg 1920

ttcgtttgtg caagcttcaa cgatgccaaa agggtataat aagcgtcatt tgcagcattg 1980

tgaagaaaac tatgtggcaa gccaagcctg cgaagaatgt accatctaat tgggcccaca 2040

caccatagct tcaaaatgtt tctactcctt ttttactctt ccagattttc tcggactccg 2100

cgcatcgccg taccacttca aaacacccaa gcacagcata ctaaatttcc cctctttctt 2160

cctctagggt gtcgttaatt acccgtacta aaggtttgga aaagaaaaaa gagaccgcct 2220

cgtttctttt tcttcgtcga aaaaggcaat aaaaattttt atcacgtttc tttttcttga 2280

aatttttttt tttagttttt ttctctttca gtgacctcca ttgatattta agttaataaa 2340

cggtcttcaa tttctcaagt ttcagtttca tttttcttgt tctattacaa ctttttttac 2400

ttcttgttca ttagaaagaa agcatagcaa tctaatctaa ggggcggtgt tgacaattaa 2460

tcatcggcat agtatatcgg catagtataa tacgacaagg tgaggaacta aagcatggct 2520

aagttgactt ccgctgttcc agttttgaca gctagagatg ttgctggtgc tgttgaattt 2580

tggactgata gattgggatt ttcaagagac ttcgttgagg atgactttgc tggtgttgtt 2640

agagatgacg ttactttgtt catttctgct gttcaagatc aggttgttcc tgacaacaca 2700

ttggcttggg tttgggttag aggtttggat gaattgtacg ctgaatggtc tgaggttgtt 2760

tccactaatt tcagagacgc ttctggacca gctatgacag aaatcggaga gcaaccttgg 2820

ggaagagagt ttgctttgag agatccagct ggaaactgtg ttcatttcgt tgctgaagag 2880

caggactaac acgtccgacg gcggcccacg ggtcccaggc ctcggagatc cgtccccctt 2940

ttcctttgtc gatatcatgt aattagttat gtcacgctta cattcacgcc ctccccccac 3000

atccgctcta accgaaaagg aaggagttag acaacctgaa gtctaggtcc ctatttattt 3060

ttttatagtt atgttagtat taagaacgtt atttatattt caaatttttc ttttttttct 3120

gtacagacgc gtgtacgcat gtaacattat actgaaaacc ttgcttgaga aggttttggg 3180

acgctcgaag gctttaattt gcaagctact agtatgtgag caaaaggcca gcaaaaggcc 3240

aggaaccgta aaaaggccgc gttgctggcg tttttccata ggctccgccc ccctgacgag 3300

catcacaaaa atcgacgctc aagtcagagg tggcgaaacc cgacaggact ataaagatac 3360

caggcgtttc cccctggaag ctccctcgtg cgctctcctg ttccgaccct gccgcttacc 3420

ggatacctgt ccgcctttct cccttcggga agcgtggcgc tttctcatag ctcacgctgt 3480

aggtatctca gttcggtgta ggtcgttcgc tccaagctgg gctgtgtgca cgaacccccc 3540

gttcagcccg accgctgcgc cttatccggt aactatcgtc ttgagtccaa cccggtaaga 3600

cacgacttat cgccactggc agcagccact ggtaacagga ttagcagagc gaggtatgta 3660

ggcggtgcta cagagttctt gaagtggtgg cctaactacg gctacactag aagaacagta 3720

tttggtatct gcgctctgct gaagccagtt accttcggaa aaagagttgg tagctcttga 3780

tccggcaaac aaaccaccgc tggtagcggt ggtttttttg tttgcaagca gcagattacg 3840

cgcagaaaaa aaggatctca agtctgacgc tcagtggaac gaaaactcac gttaagggga 3900

attccctttg atcttttcta cggggtctga cgctcagtgg aacgaaaaat aacttcgtat 3960

agcatacatt atacgaagtt atataaacaa ataggggttc cgcgcacatt tccccgaaaa 4020

gtgccacctg gg 4032

<210> 35
<211> 3749
<212> DNA
<213> Artificial Sequence

<220>
<223> pBiPACE

<400> 35
catggcacac catcaccacc atcacagcag cggtaccgtc gacccgactg gaaagcgggc 60

agtgagcgca acgcaattaa tgtgagttag ctcactcatt aggcacccca ggctttacac 120

tttatgcttc cggctcgtat gttgtgtgga attgtgagcg gataacaatt tcacacagga 180

aacagctatg accatgatta cggattcact ggccgtcgtt ttacaacgtc gtgactggga 240

aaaccctggc gttacccaac ttaatcgcct tgcagcacat ccccctttcg ccagctggcg 300

taatagcgaa gaggcccgca ccgatcgccc ttcccaacag ttgcgcagcc tgaatggcga 360

atggcgccaa gctttctaga ccagtttaaa catcaccatc accatcatca tcaccatcac 420

taatggccat caagaggatg tcagaatgcc atttgcctga gagatgcagg cttcattttt 480

gatacttttt tatttgtaac ctatatagta taggattttt tttgtcattt tgtttcttct 540

cgtacgagct tgctcctgat cagcctatct cgcagctgat gaatatcttg tggtaggggt 600

ttgggaaaat cattcgagtt tgatgttttt cttggtattt cccactcctc ttcagagtac 660

agaagattaa gtgacacgtt cgtttgtgca agcttcaacg atgccaaaag ggtataataa 720

gcgtcatttg cagcattgtg aagaaaacta tgtggcaagc caagcctgcg aagaatgtac 780

catctaattg ggcccacaca ccatagcttc aaaatgtttc tactcctttt ttactcttcc 840

agattttctc ggactccgcg catcgccgta ccacttcaaa acacccaagc acagcatact 900

aaatttcccc tctttcttcc tctagggtgt cgttaattac ccgtactaaa ggtttggaaa 960

agaaaaaaga gaccgcctcg tttctttttc ttcgtcgaaa aaggcaataa aaatttttat 1020

cacgtttctt tttcttgaaa tttttttttt tagttttttt ctctttcagt gacctccatt 1080

gatatttaag ttaataaacg gtcttcaatt tctcaagttt cagtttcatt tttcttgttc 1140

tattacaact ttttttactt cttgttcatt agaaagaaag catagcaatc taatctaagg 1200

ggcggtgttg acaattaatc atcggcatag tatatcggca tagtataata cgacaaggtg 1260

aggaactaaa gcatggctaa gttgacttcc gctgttccag ttttgacagc tagagatgtt 1320

gctggtgctg ttgaattttg gactgataga ttgggatttt caagagactt cgttgaggat 1380

gactttgctg gtgttgttag agatgacgtt actttgttca tttctgctgt tcaagatcag 1440

gttgttcctg acaacacatt ggcttgggtt tgggttagag gtttggatga attgtacgct 1500

gaatggtctg aggttgtttc cactaatttc agagacgctt ctggaccagc tatgacagaa 1560

atcggagagc aaccttgggg aagagagttt gctttgagag atccagctgg aaactgtgtt 1620

catttcgttg ctgaagagca ggactaacac gtccgacggc ggcccacggg tcccaggcct 1680

cggagatccg tccccctttt cctttgtcga tatcatgtaa ttagttatgt cacgcttaca 1740

ttcacgccct ccccccacat ccgctctaac cgaaaaggaa ggagttagac aacctgaagt 1800

ctaggtccct atttattttt ttatagttat gttagtatta agaacgttat ttatatttca 1860

aatttttctt ttttttctgt acagacgcgt gtacgcatgt aacattatac tgaaaacctt 1920

gcttgagaag gttttgggac gctcgaaggc tttaatttgc aagctactag tatgtgagca 1980

aaaggccagc aaaaggccag gaaccgtaaa aaggccgcgt tgctggcgtt tttccatagg 2040

ctccgccccc ctgacgagca tcacaaaaat cgacgctcaa gtcagaggtg gcgaaacccg 2100

acaggactat aaagatacca ggcgtttccc cctggaagct ccctcgtgcg ctctcctgtt 2160

ccgaccctgc cgcttaccgg atacctgtcc gcctttctcc cttcgggaag cgtggcgctt 2220

tctcatagct cacgctgtag gtatctcagt tcggtgtagg tcgttcgctc caagctgggc 2280

tgtgtgcacg aaccccccgt tcagcccgac cgctgcgcct tatccggtaa ctatcgtctt 2340

gagtccaacc cggtaagaca cgacttatcg ccactggcag cagccactgg taacaggatt 2400

agcagagcga ggtatgtagg cggtgctaca gagttcttga agtggtggcc taactacggc 2460

tacactagaa gaacagtatt tggtatctgc gctctgctga agccagttac cttcggaaaa 2520

agagttggta gctcttgatc cggcaaacaa accaccgctg gtagcggtgg tttttttgtt 2580

tgcaagcagc agattacgcg cagaaaaaaa ggatctcaag tctgacgctc agtggaacga 2640

aaactcacgt taaggggaat tccctttgat cttttctacg gtcgagataa gctgggggaa 2700

cattcgcgaa aatgaaacaa gtcggctgtt atagtatatt tattataata ttgaaagatc 2760

tcaaaagact acttattttt gaatgaacca agtatgaaat caacctattt ggggttgacc 2820

aaaataagta aatattaatt gtcgaggtct gacgctcagt ggaacgaaaa ataacttcgt 2880

atagcataca ttatacgaag ttatataaac aaataggggt tccgcgcaca tttccccgaa 2940

aagtgccacc tgggatgcat aactataacg gtcctaaggt agcgacctag gagatctttt 3000

ttgtagaaat gtcttggtgt cctcgtccaa tcaggtagcc atctctgaaa tatctggctc 3060

cgttgcaact ccgaacgacc tgctggcaac gtaaaattct ccggggtaaa acttaaatgt 3120

ggagtaatgg aaccagaaac gtctcttccc ttctctctcc ttccaccgcc cgttaccgtc 3180

cctaggaaat tttactctgc tggagagctt cttctacggc ccccttgcag caatgctctt 3240

cccagcatta cgttgcgggt aaaacggagg tcgtgtaccc gacctagcag cccagggatg 3300

gaaaagtccc ggccgtcgct ggcaataata gcgggcggac gcatgtcatg agattattgg 3360

aaaccaccag aatcgaatat aaaaggcgaa cacctttccc aattttggtt tctcctgacc 3420

caaagacttt aaatttaatt tatttgtccc tatttcaatc aattgaacaa ctatttcgaa 3480

acgatgagat ttccatctat tttcacagct gttttgtttg ctgcttcttc cgctttggct 3540

gctccagtta acactacaac tgaagatgag actgctcaaa ttcctgctga agctgttatc 3600

ggttacttgg acttggaggg agattttgac gttgctgttt tgcctttctc aaactctaca 3660

aacaacggtt tgttgttcat caacacaact atcgcttcca tcgctgctaa ggaagaggga 3720

gtttcattgg ataaaagaga agctgaggc 3749

<210> 36
<211> 3764
<212> DNA
<213> Artificial Sequence

<220>
<223> pPDK

<400> 36
ctatgtcggg tgcggagaaa gaggtaatga aatggctaga gatctaacat ccaaagacga 60

aaggttgaat gaaacctttt tgccatccga catccacagg tccattctca cacataagtg 120

ccaaacgcaa caggagggga tacactagca gcagaccgtt gcaaacgcag gacctccact 180

cctcttctcc tcaacaccca cttttgccat cgaaaaacca gcccagttat tgggcttgat 240

tggagctagc tcattccaat tccttctatt aggctactaa caccatgact ttattagcct 300

gtctatcctg gcccccctgg cgaggttcat gtttgtttat ttccgaatgc aacaagctcc 360

gcattacacc cgaacatcac tccagatgag ggctttctga gtgtggggtc aaatagtttc 420

atgttcccca aatggcccaa aactgacagt ttaaacgctg tcttggaacc taatatgaca 480

aaagcgtgat ctcatccaag atgaactaag tttggttcgt tgaaatgcta acggccagtt 540

ggtcaaaaag aaacttccaa aagtcggcat accgtttgtc ttgtttggta ttgattgacg 600

aatgctcaaa aataatctca ttaatgctta gcgcagtctc tctatcgctt ctgaaccccg 660

gtgcacctgt gccgaaacgc aaatggggaa acacccgctt tttggatgat tatgcattgt 720

ctccacattg tatgcttcct agattctggt gggaatactg ctgatagcct aacgttcatg 780

atcaaaattt aactgttcta acccctactt gacagcaata tataaacaga aggaagctgc 840

cctgtcttaa accttttttt ttatcatcat tattagctta ctttcataat tgcgactggt 900

tccaattgac aagcttttga ttttaacgac ttttaacgac aacttgagaa gatcaaaaaa 960

caactaatta ttcgaaacga tgagatttcc atctattttc acagctgttt tgtttgctgc 1020

ttcttccgct ttggctgctc cagttaacac tacaactgaa gatgagactg ctcaaattcc 1080

tgctgaagct gttatcggtt acttggactt ggagggagat tttgacgttg ctgttttgcc 1140

tttctcaaac tctacaaaca acggtttgtt gttcatcaac acaactatcg cttccatcgc 1200

tgctaaggaa gagggagttt cattggataa aagagaagct gaggccatgg cacaccatca 1260

ccaccatcac agcagcggta ccgtcgaccc gactggaaag cgggcagtga gcgcaacgca 1320

attaatgtga gttagctcac tcattaggca ccccaggctt tacactttat gcttccggct 1380

cgtatgttgt gtggaattgt gagcggataa caatttcaca caggaaacag ctatgaccat 1440

gattacggat tcactggccg tcgttttaca acgtcgtgac tgggaaaacc ctggcgttac 1500

ccaacttaat cgccttgcag cacatccccc tttcgccagc tggcgtaata gcgaagaggc 1560

ccgcaccgat cgcccttccc aacagttgcg cagcctgaat ggcgaatggc gccaagcttt 1620

ctagaccagt ttaaacatca ccatcaccat catcatcacc atcactaatg gccatcaaga 1680

ggatgtcaga atgccatttg cctgagagat gcaggcttca tttttgatac ttttttattt 1740

gtaacctata tagtatagga ttttttttgt cattttgttt cttctcgtac gagcttgctc 1800

ctgatcagcc tatctcgcag ctgatgaata tcttgtggta ggggtttggg aaaatcattc 1860

gagtttgatg tttttcttgg tatttcccac tcctcttcag agtacagaag attaagtgac 1920

acgttcgttt gtgcaagctt caacgatgcc aaaagggtat aataagcgtc atttgcagca 1980

ttgtgaagaa aactatgtgg caagccaagc ctgcgaagaa tgtaccatgt gcctggcaga 2040

taacttcgta taatgtatgc tatacgaagt tatggtacgt actaagctct catgtttcac 2100

gtactaagct ctcatgttta acgtactaag ctctcatgtt taacgaacta aaccctcatg 2160

gctaacgtac taagctctca tggctaacgt actaagctct catgtttcac gtactaagct 2220

ctcatgtttg aacaataaaa ttaatataaa tcagcaactt aaatagcctc taaggtttta 2280

agttttataa gaaaaaaaag aatatataag gcttttaaag cttttaaggt ttaacggttg 2340

tggacaacaa gccagggatg taacgcactg agaagccctt agagcctctc aaagcaattt 2400

tcagtgacac aggaacactt aacggctgac agaattagct tcacgctgcc gcaagcactc 2460

agggcgcaag ggctgctaaa ggaagcggaa cacgtagaaa gccagtccgc agaaacggtg 2520

ctgaccccgg atgaatgtca gctactgggc tatctggaca agggaaaacg caagcgcaaa 2580

gagaaagcag gtagcttgca gtgggcttac atggcgatag ctagactggg cggttttatg 2640

gacagcaagc gaaccggaat tgccagctgg ggcgccctct ggtaaggttg ggaagccctg 2700

caaagtaaac tggatggctt tcttgccgcc aaggatctga tggcgcaggg gatcaagatc 2760

tgatcaagag acaggatgag gatcgtttcg catgattgaa caagatggat tgcacgcagg 2820

ttctccggcc gcttgggtgg agaggctatt cggctatgac tgggcacaac agacaatcgg 2880

ctgctctgat gccgccgtgt tccggctgtc agcgcagggg cgcccggttc tttttgtcaa 2940

gaccgacctg tccggtgccc tgaatgaact gcaggacgag gcagcgcggc tatcgtggct 3000

ggccacgacg ggcgttcctt gcgcagctgt gctcgacgtt gtcactgaag cgggaaggga 3060

ctggctgcta ttgggcgaag tgccggggca ggatctcctg tcatctcacc ttgctcctgc 3120

cgagaaagta tccatcatgg ctgatgcaat gcggcggctg catacgcttg atccggctac 3180

ctgcccattc gaccaccaag cgaaacatcg catcgagcga gcacgtactc ggatggaagc 3240

cggtcttgtc gatcaggatg atctggacga agagcatcag gggctcgcgc cagccgaact 3300

gttcgccagg ctcaaggcgc gcatgcccga cggcgaggat ctcgtcgtga cacatggcga 3360

tgcctgcttg ccgaatatca tggtggaaaa tggccgcttt tctggattca tcgactgtgg 3420

ccggctgggt gtggcggacc gctatcagga catagcgttg gctacccgtg atattgctga 3480

agagcttggc ggcgaatggg ctgaccgctt cctcgtgctt tacggtatcg ccgctcccga 3540

ttcgcagcgc atcgccttct atcgccttct tgacgagttc ttctgagcgg gactctgggg 3600

ttcgaaatga ccgaccaagc gacgcccaac ctgccatcac gagatttcga ttccaccgcc 3660

gccttctatg aaaggttggg cttcggaatc gttttccggg acgccggctg gatgatcctc 3720

cagcgcgggg atctcatgct ggagttcttc gcccaccccg ggat 3764

<210> 37

<211>  3702
<212>  DNA
<213>  Artificial Sequence

<220>
<223>  pPDC

<400>  37
atcaacgtct cattttcgcc aaaagttggc ccagatctat gtcgggtgcg gagaaagagg      60

taatgaaatg gctagagatc taacatccaa agacgaaagg ttgaatgaaa cctttttgcc      120

atccgacatc cacaggtcca ttctcacaca taagtgccaa acgcaacagg aggggataca      180

ctagcagcag accgttgcaa acgcaggacc tccactcctc ttctcctcaa cacccacttt      240

tgccatcgaa aaaccagccc agttattggg cttgattgga gctagctcat tccaattcct      300

tctattaggc tactaacacc atgactttat tagcctgtct atcctggccc ccctggcgag      360

gttcatgttt gtttatttcc gaatgcaaca agctccgcat tacacccgaa catcactcca      420

gatgagggct ttctgagtgt ggggtcaaat agtttcatgt tccccaaatg gcccaaaact      480

gacagttata acgctgtctt ggaacctaat atgacaaaag cgtgatctca tccaagatga      540

actaagtttg gttcgttgaa atgctaacgg ccagttggtc aaaaagaaac ttccaaaagt      600

cggcataccg tttgtcttgt ttggtattga ttgacgaatg ctcaaaaata atctcattaa      660

tgcttagcgc agtctctcta tcgcttctga accccggtgc acctgtgccg aaacgcaaat      720

ggggaaacac ccgctttttg gatgattatg cattgtctcc acattgtatg cttcctagat      780

tctggtggga atactgctga tagcctaacg ttcatgatca aaatttaact gttctaaccc      840

ctacttgaca gcaatatata aacagaagga agctgccctg tcttaaacct ttttttttat      900

catcattatt agcttacttt cataattgcg actggttcca attgacaagc ttttgatttt      960

aacgactttt aacgacaact tgagaagatc aaaaaacaac taattattcg aaacgatgag      1020

atttccatct attttcacag ctgttttgtt tgctgcttct tccgctttgg ctgctccagt      1080

taacactaca actgaagatg agactgctca aattcctgct gaagctgtta tcggttactt      1140

ggacttggag ggagattttg acgttgctgt tttgcctttc tcaaactcta caaacaacgg      1200

tttgttgttc atcaacacaa ctatcgcttc catcgctgct aaggaagagg gagtttcatt      1260

ggataaaaga gaagctgagg ccatggcaca ccatcaccac catcacagca gcggtaccgt      1320

cgacccgact ggaaagcggg cagtgagcgc aacgcaatta atgtgagtta gctcactcat      1380

taggcacccc aggctttaca ctttatgctt ccggctcgta tgttgtgtgg aattgtgagc      1440

ggataacaat ttcacacagg aaacagctat gaccatgatt acggattcac tggccgtcgt      1500

tttacaacgt cgtgactggg aaaaccctgg cgttacccaa cttaatcgcc ttgcagcaca      1560

tccccctttc gccagctggc gtaatagcga agaggcccgc accgatcgcc cttcccaaca      1620

gttgcgcagc ctgaatggcg aatggcgcca agctttctag accagtttaa acatcaccat      1680

caccatcatc atcaccatca ctaatggcca tcaagaggat gtcagaatgc catttgcctg 1740

agagatgcag gcttcatttt tgatactttt ttatttgtaa cctatatagt ataggatttt 1800

ttttgtcatt ttgtttcttc tcgtacgagc ttgctcctga tcagcctatc tcgcagctga 1860

tgaatatctt gtggtagggg tttgggaaaa tcattcgagt ttgatgtttt tcttggtatt 1920

tcccactcct cttcagagta cagaagatta agtgacacgt tcgtttgtgc aagcttcaac 1980

gatgccaaaa gggtataata agcgtcattt gcagcattgt gaagaaaact atgtggcaag 2040

ccaagcctgc gaagaatgta ccatgtgcct ggcagataac ttcgtataat gtatgctata 2100

cgaagttatg gtaccgcggc cgcgtagagg atctgttgat cagcagttca acctgttgat 2160

agtacgtact aagctctcat gtttcacgta ctaagctctc atgtttaacg tactaagctc 2220

tcatgtttaa cgaactaaac cctcatggct aacgtactaa gctctcatgg ctaacgtact 2280

aagctctcat gtttcacgta ctaagctctc atgtttgaac aataaaatta atataaatca 2340

gcaacttaaa tagcctctaa ggttttaagt tttataagaa aaaaaagaat atataaggct 2400

tttaaagctt ttaaggttta acggttgtgg acaacaagcc agggatgtaa cgcactgaga 2460

agcccttaga gcctctcaaa gcaattttga gtgacacagg aacacttaac ggctgacaga 2520

attagcttca cgctgccgca agcactcagg gcgcaagggc tgctaaagga agcggaacac 2580

gtagaaagcc agtccgcaga aacggtgctg accccggatg aatgtcagct gggaggcaga 2640

ataaatgatc atatcgtcaa ttattacctc cacggggaga gcctgagcaa actggcctca 2700

ggcatttgag aagcacacgg tcacactgct tccggtagtc aataaaccgg taaaccagca 2760

atagacataa gcggctattt aacgaccctg ccctgaaccg acgaccgggt cgaatttgct 2820

ttcgaatttc tgccattcat ccgcttatta tcacttattc aggcgtagca accaggcgtt 2880

taagggcacc aataactgcc ttaaaaaaat tacgccccgc cctgccactc atcgcagtac 2940

tgttgtaatt cattaagcat tctgccgaca tggaagccat cacaaacggc atgatgaacc 3000

tgaatcgcca gcggcatcag caccttgtcg ccttgcgtat aatatttgcc catggtgaaa 3060

acgggggcga agaagttgtc catattggcc acgtttaaat caaaactggt gaaactcacc 3120

cagggattgg ctgagacgaa aaacatattc tcaataaacc ctttagggaa ataggccagg 3180

ttttcaccgt aacacgccac atcttgcgaa tatatgtgta gaaactgccg gaaatcgtcg 3240

tggtattcac tccagagcga tgaaaacgtt tcagtttgct catggaaaac ggtgtaacaa 3300

gggtgaacac tatcccatat caccagctca ccgtctttca ttgccatacg gaattccgga 3360

tgagcattca tcaggcgggc aagaatgtga ataaaggccg gataaaactt gtgcttattt 3420

ttctttacgg tctttaaaaa ggccgtaata tccagctgaa cggtctggtt ataggtacat 3480

tgagcaactg actgaaatgc ctcaaaatgt tctttacgat gccattggga tatatcaacg 3540

gtggtatatc cagtgatttt tttctccatt ttagcttcct tagctcctga aaatctcgat 3600

aactcaaaaa atacgcccgg tagtgatctt atttcattat ggtgaaagtt ggaccctctt 3660

acgtgccgat caacgtctca ttttcgccaa aagttggccc ag 3702

<210> 38
<211> 3714
<212> DNA
<213> Artificial Sequence

<220>
<223> pPDS

<400> 38
ctatgtcggg tgcggagaaa gaggtaatga aatggctaga gatctaacat ccaaagacga 60

aaggttgaat gaaacctttt tgccatccga catccacagg tccattctca cacataagtg 120

ccaaacgcaa caggagggga tacactagca gcagaccgtt gcaaacgcag gacctccact 180

cctcttctcc tcaacaccca cttttgccat cgaaaaacca gcccagttat tgggcttgat 240

tggagctagc tcattccaat tccttctatt aggctactaa caccatgact ttattagcct 300

gtctatcctg gcccccctgg cgaggttcat gtttgtttat ttccgaatgc aacaagctcc 360

gcattacacc cgaacatcac tccagatgag ggctttctga gtgtggggtc aaatagtttc 420

atgttcccca aatggcccaa aactgacagt ttaaacgctg tcttggaacc taatatgaca 480

aaagcgtgat ctcatccaag atgaactaag tttggttcgt tgaaatgcta acggccagtt 540

ggtcaaaaag aaacttccaa aagtcggcat accgtttgtc ttgtttggta ttgattgacg 600

aatgctcaaa aataatctca ttaatgctta gcgcagtctc tctatcgctt ctgaaccccg 660

gtgcacctgt gccgaaacgc aaatggggaa acacccgctt tttggatgat tatgcattgt 720

ctccacattg tatgcttcct agattctggt gggaatactg ctgatagcct aacgttcatg 780

atcaaaattt aactgttcta acccctactt gacagcaata tataaacaga aggaagctgc 840

cctgtcttaa accttttttt ttatcatcat tattagctta ctttcataat tgcgactggt 900

tccaattgac aagcttttga ttttaacgac ttttaacgac aacttgagaa gatcaaaaaa 960

caactaatta ttcgaaacga tgagatttcc atctattttc acagctgttt tgtttgctgc 1020

ttcttccgct ttggctgctc cagttaacac tacaactgaa gatgagactg ctcaaattcc 1080

tgctgaagct gttatcggtt acttggactt ggagggagat tttgacgttg ctgttttgcc 1140

tttctcaaac tctacaaaca acggtttgtt gttcatcaac acaactatcg cttccatcgc 1200

tgctaaggaa gagggagttt cattggataa aagagaagct gaggccatgg cacaccatca 1260

ccaccatcac agcagcggta ccgtcgaccc gactggaaag cgggcagtga gcgcaacgca 1320

attaatgtga gttagctcac tcattaggca ccccaggctt tacactttat gcttccggct 1380

cgtatgttgt gtggaattgt gagcggataa caatttcaca caggaaacag ctatgaccat 1440

gattacggat tcactggccg tcgttttaca acgtcgtgac tgggaaaacc ctggcgttac 1500

ccaacttaat cgccttgcag cacatccccc tttcgccagc tggcgtaata gcgaagaggc 1560

ccgcaccgat cgcccttccc aacagttgcg cagcctgaat ggcgaatggc gccaagcttt 1620

ctagaccagt ttaaacatca ccatcaccat catcatcacc atcactaatg gccatcaaga 1680

ggatgtcaga atgccatttg cctgagagat gcaggcttca tttttgatac ttttttattt 1740

gtaacctata tagtatagga tttttttttgt catttgtttt cttctcgtac gagcttgctc 1800

ctgatcagcc tatctcgcag ctgatgaata tcttgtggta ggggtttggg aaaatcattc 1860

gagtttgatg tttttcttgg tatttcccac tcctcttcag agtacagaag attaagtgac 1920

acgttcgttt gtgcaagctt caacgatgcc aaaagggtat aataagcgtc atttgcagca 1980

ttgtgaagaa aactatgtgg caagccaagc ctgcgaagaa tgtaccatgt gcctggcaga 2040

taacttcgta taatgtatgc tatacgaagt tatggtacgt actaagctct catgtttcac 2100

gtactaagct ctcatgttta acgtactaag ctctcatgtt taacgaacta aaccctcatg 2160

gctaacgtac taagctctca tggctaacgt actaagctct catgtttcac gtactaagct 2220

ctcatgtttg aacaataaaa ttaatataaa tcagcaactt aaatagcctc taaggtttta 2280

agttttataa gaaaaaaaag aatatataag gcttttaaag cttttaaggt ttaacggttg 2340

tggacaacaa gccagggatg taacgcactg agaagccctt agagcctctc aaagcaattt 2400

tgagtgacac aggaacactt aacggctgac ataattcagc ttcacgctgc cgcaagcact 2460

cagggcgcaa gggctgctaa aggaagcgga acacgtagaa agccagtccg cagaaacggt 2520

gctgaccccg gatgaatgtc agctgggagg cagaataaat gatcatatcg tcaattatta 2580

cctccacggg gagagcctga gcaaactggc ctcaggcatt tgagaagcac acggtcacac 2640

tgcttccggt agtcaataaa ccggtaagta gcgtatgcgc tcacgcaact ggtccagaac 2700

cttgaccgaa cgcagcggtg gtaacggcgc agtggcggtt ttcatggctt gttatgactg 2760

tttttttggg gtacagtcta tgcctcgggc atccaagcag caagcgcgtt acgccgtggg 2820

tcgatgtttg atgttatgga gcagcaacga tgttacgcag cagggcagtc gccctaaaac 2880

aaagttaaac atcatgaggg aagcggtgat cgccgaagta tcgactcaac tatcagaggt 2940

agttggcgtc atcgagcgcc atctcgaacc gacgttgctg gccgtacatt tgtacggctc 3000

cgcagtggat ggcggcctga agccacacag tgatattgat ttgctggtta cggtgaccgt 3060

aaggcttgat gaaacaacgc ggcgagcttt gatcaacgac cttttggaaa cttcggcttc 3120

ccctggagag agcgagattc tccgcgctgt agaagtcacc attgttgtgc acgacgacat 3180

cattccgtgg cgttatccag ctaagcgcga actgcaattt ggagaatggc agcgcaatga 3240

cattcttgca ggtatcttcg agccagccac gatcgacatt gatctggcta tcttgctgac 3300

aaaagcaaga gaacatagcg ttgccttggt aggtccagcg gcggaggaac tctttgatcc 3360

ggttcctgaa caggatctat ttgaggcgct aaatgaaacc ttaacgctat ggaactcgcc 3420

gcccgactgg gctggcgatg agcgaaatgt agtgcttacg ttgtcccgca tttggtacag 3480

cgcagtaacc ggcaaaatcg cgccgaagga tgtcgctgcc gactgggcaa tggagcgcct 3540

gccggcccag tatcagcccg tcatacttga agctagacag gcttatcttg gacaagaaga 3600

agatcgcttg gcctcgcgcg cagatcagtt ggaagaattt gtccactacg tgaaaggcga 3660

gatcaccaag gtagtcggca aataatgtct aacaattcgt tcaagccgac ggat 3714

<210> 39
<211> 26
<212> DNA
<213> Artificial Sequence

<220>
<223> sAOX1InsFor primer

<400> 39
cattggataa aagagaagct gaggcc 26

<210> 40
<211> 29
<212> DNA
<213> Artificial Sequence

<220>
<223> icAOX1InsFor primer

<400> 40
caaaaaacaa ctaattattc gaaacgatg 29

<210> 41
<211> 25
<212> DNA
<213> Artificial Sequence

<220>
<223> AOX1InsRev primer

<400> 41
gatgatgatg gtgatggtga tgttt 25

<210> 42
<211> 25
<212> DNA
<213> Artificial Sequence

<220>
<223> AOX1VecFor primer

<400> 42
aaacatcacc atcaccatca tcatc 25

<210>  43
<211>  26
<212>  DNA
<213>  Artificial Sequence

<220>
<223>  sAOX1VecRev

<400>  43
ggcctcagct tctcttttat ccaatg                                            26


<210>  44
<211>  29
<212>  DNA
<213>  Artificial Sequence

<220>
<223>  icAOX1VecRev primer

<400>  44
   catcgtttcg aataattagt tgttttttg                                          29


<210>  45
<211>  28
<212>  DNA
<213>  Artificial Sequence

<220>
<223>  ttAOX1InsVecFor primer

<400>  45
aaacatcacc atcaccatca tcatcacc                                          28


<210>  46
<211>  50
<212>  DNA
<213>  Artificial Sequence

<220>
<223>  ttAOX1VecRev primer

<400>  46
ggaccgttat agttatgcat ttagatggta cattcttcgc aggcttggct                  50


<210>  47
<211>  52
<212>  DNA
<213>  Artificial Sequence

<220>
<223>  pAOX1VecFor promer

<400>  47
cctgcgaaga atgtaccatc taaatgcata actataacgg tcctaaggta gc               52


<210>  48
<211>  34
<212>  DNA

<213>  Artificial Sequence

<220>
<223>  pAOX1VecInsRe primer

<400>  48
cgtttcgaat aattagttgt tttttgatct tctc                                    34


<210>  49
<211>  34
<212>  DNA
<213>  Artificial Sequence

<220>
<223>  LoxP imperfect repeat

<400>  49
ataacttcgt atagcataca ttatacgaag ttat                                    34


<210>  50
<211>  267
<212>  DNA
<213>  Artificial Sequence

<220>
<223>  engineered alpha-mating factor, alphaMF

<400>  50
atgagatttc catctatttt cacagctgtt ttgtttgctg cttcttccgc tttggctgct        60

ccagttaaca ctacaactga agatgagact gctcaaattc ctgctgaagc tgttatcggt       120

tacttggact tggagggaga ttttgacgtt gctgttttgc ctttctcaaa ctctacaaac       180

aacggtttgt tgttcatcaa cacaactatc gcttccatcg ctgctaagga agagggagtt       240

tcattggata aaagagaagc tgaggcc                                           267


<210>  51
<211>  47
<212>  DNA
<213>  Artificial Sequence

<220>
<223>  icAOX1InsForHPV16L1 primer

<400>  51
caaaaaacaa ctaattattc gaaacgatgt ccttgtggtt gccatca                      47


<210>  52
<211>  51
<212>  DNA
<213>  Artificial Sequence

<220>
<223>  AOX1InsRevHPV16L1 primer

<400>  52
gatgatgatg gtgatggtga tgtttaagtt ttctcttttt acgtttagcg g                 51

<210>  53
<211>  46
<212>  DNA
<213>  Artificial Sequence

<220>
<223>  icAOX1InsForHPV18L1 primer

<400>  53
caaaaaacaa ctaattattc gaaacgatgg cactttggcg tcctag                    46


<210>  54
<211>  46
<212>  DNA
<213>  Artificial Sequence

<220>
<223>  InsRevHPV18L1 primer

<400>  54
gatgatgatg gtgatggtga tgttttctag cacgaactct gactct                    46


<210>  55
<211>  52
<212>  DNA
<213>  Artificial Sequence

<220>
<223>  icAOX1InsForPS primer

<400>  55
caaaaaacaa ctaattattc gaaacgatgt ctcaccatca ccaccatcac ag             52


<210>  56
<211>  51
<212>  DNA
<213>  Artificial Sequence

<220>
<223>  AOX1InsRevPS primer

<400>  56
gatgatgatg gtgatggtga tgtttttagt tagagtgtgc tttagcaaag g              51


<210>  57
<211>  975
<212>  DNA
<213>  Schizosaccharomyces pombe


<220>
<221>  PSAT
<222>  (1)..(975)

<400>  57
atggacgccc ccaggcaggt ggtcaacttt gggcctggtc ccgccaagct gccgcactca     60

gtgttgttag agatacaaaa ggaattatta gactacaaag gagttggcat tagtgttctt 120

gaaatgagtc acaggtcatc agattttgcc aagattatta acaatacaga gaatcttgtg 180

cgggaattgc tagctgttcc agacaactat aaggtgattt ttctgcaagg aggtgggtgc 240

ggccagttca gtgctgtccc cttaaacctc attggcttga aagcaggaag gtgtgcggac 300

tatgtggtga caggagcttg gtcagctaag gccgcagaag aagccaagaa gtttgggact 360

ataaatatcg ttcaccctaa acttgggagt tatacaaaaa ttccagatcc aagcacctgg 420

aacctcaacc cagatgcctc ctacgtgtat tattgcgcaa atgagacggt gcatggtgtg 480

gagtttgact ttatacccga tgtcaaggga gcagtactgg tttgtgacat gtcctcaaac 540

ttcctgtcca agccagtgga tgtttccaag tttggtgtga tttttgctgg tgcccagaag 600

aatgttggct ctgctggggt caccgtggtg attgtccgtg atgacctgct ggggtttgcc 660

ctccgagagt gcccctcggt cctggaatac aaggtgcagg ctggaaacag ctccttgtac 720

aacacgcctc catgtttcag catctacgtc atgggcttgg ttctggagtg gattaaaaac 780

aatggaggtg ccgcggccat ggagaagctt agctccatca aatctcaaac aatttatgag 840

attattgata attctcaagg attctacgtg tctgtgggag gcatccgggc ctctctgtat 900

aatgctgtca caattgaaga cgttcagaag ctggccgcct tcatgaaaaa atttttggag 960

atgcatcagc tatga 975

<210> 58
<211> 750
<212> DNA
<213> Artificial Sequence

<220>
<223> a-MUC1 IIB6 scFv - His8

<400> 58
caaatgcaac ttgttcaatc tggagctgaa gtgaaaaagc ctggtgctag cgttaaagtg 60

tcatgtaaag cctccggata taccttcact ggtcattaca tgcactgggt ccgtcaagct 120

ccaggacagg gtctggaatg gatgggctgg attgaccccg taactggagg tacaaagtat 180

gctcagaact ttcaaggttg ggttacgatg actagagaca cgtctatccg tactgcttac 240

atggagctgt ctaggttacg atcagacgat acagcaatgt attactgtgc tcgtgaagta 300

actggtgata ggggtcaatt cgataaatgg ggtcagggta ctctagttac tgttgcttct 360

gcatccacta aaggtccatc ggttaagctc gaagagggag aatttagtga ggcaagagtg 420

caaagtgttt tgacacagcc accggtgagc gtggcccctg gtaagacagc tagaattacc 480

tgtgggggaa ataacattgg ttccaagtca gtccattggt atcagcaaaa acctggacaa 540

gccccagtct tggttatcta ctatgattcc gacagaccat caggcatccc agagagattt 600

tccggttcga atagcggaaa tacagctaca ttgaccataa gtagagtcga ggctggtgat 660

gaagcagact actactgcgg tcaacctaag gcagcaccat ctgtaacatt gttccctccc 720

tcccatcatc accatcacca ccatcactaa 750

<210> 59
<211> 918
<212> DNA
<213> Pichia pastoris

<220>
<221> HAC1
<222> (1)..(918)

<400> 59
atgcccgtag attcttctca taagacagct agcccacttc cacctcgtaa aagagcaaag 60

acggaagaag aaaaggagca gcgtcgagtg gaacgtatcc tacgtaatag gagagcggcc 120

catgcttcca gagagaagaa acgaagacac gttgaatttc tggaaaacca cgtcgtcgac 180

ctggaatctg cacttcaaga atcagccaaa gccactaaca agttgaaaga aatacaagat 240

atcattgttt caaggttgga agccttaggt ggtaccgtct cagatttgga tttaacagtt 300

ccggaagtcg attttcccaa atcttctgat ttggaaccca tgtctgatct ctcaacttct 360

tcgaaatcgg agaaagcatc tacatccact cgcagatctt tgactgagga tctggacgaa 420

gatgacgtcg ctgaatatga cgacgaagaa gaggacgaag agttacccag gaaaatgaaa 480

gtcttaaacg acaaaaacaa gagcacatct atcaagcagg agaagttgaa tgaacttcca 540

tctcctttgt catccgattt ttcagacgta gatgaagaaa agtcaactct cacacattta 600

aagttgcaac agcaacaaca acaaccagta gacaattatg tttctactcc tttgagtctt 660

ccggaggatt cagttgattt tattaaccca ggtaacttaa aaatagagtc cgatgagaac 720

ttcttgttga gttcaaatac tttacaaata aaacacgaaa atgacaccga ctacattact 780

acagctccat caggttccat caatgatttt tttaattctt atgacattag cgagtcgaat 840

cggttgcatc atccagcagc agcaccattt accgctaatg catttgattt aaatgacttt 900

gtattcttcc aggaatag 918

<210> 60
<211> 2231
<212> DNA
<213> Artificial Sequence

<220>
<223> His6 - hFB

<400> 60
ggttctcatc atcatcatca tcatggttct actccatggt ctttggcctg gccccaggga 60

tcctgctctc tggagggggt agagatcaaa ggcggctcct tccgacttct ccaagagggc 120

caggcactgg agtacgtgtg tccttctggc ttctacccgt accctgtgca gacacgtacc 180

tgcagatcta cggggtcctg gagcaccctg aagactcaag accaaaagac tgtcaggaag 240

gcagagtgca gagcaatcca ctgtccaaga ccacacgact tcgagaacgg ggaatactgg 300

ccccggtctc cctactacaa tgtgagtgat gagatctctt tccactgcta tgacggttac 360

actctccggg gctctgccaa tcgcacctgc caagtgaatg gccggtggag tgggcagaca 420

gcgatctgtg acaacggagc ggggtactgc tccaacccgg gcatccccat tggcacaagg 480

aaggtgggca gccagtaccg ccttgaagac agcgtcacct accactgcag ccgggggctt 540

accctgcgtg gctcccagcg gcgaacgtgt caggaaggtg gctcttggag cgggacggag 600

ccttcctgcc aagactcctt catgtacgac acccctcaag aggtggccga agctttcctg 660

tcttccctga cagagaccat agaaggagtc gatgctgagg atgggcacgg cccaggggaa 720

caacagaagc ggaagatcgt cctggaccct tcaggctcca tgaacatcta cctggtgcta 780

gatggatcag gcagcattgg ggccagcaac ttcacaggag ccaaaaagtg tctagtcaac 840

ttaattgaga aggtggcaag ttatggtgtg aagccaagat atggtctagt gacatatgcc 900

acatacccca aaatttgggt caaagtgtct gaagcagaca gcagtaatgc agactgggtc 960

acgaagcagc tcaatgaaat caattatgaa gaccacaagt tgaagtcagg gactaacacc 1020

aagaaggccc tccaggcagt gtacagcatg atgagctggc cagatgacgt ccctcctgaa 1080

ggctggaacc gcacccgcca tgtcatcatc ctcatgactg atggattgca caacatgggc 1140

ggggacccaa ttactgtcat tgatgagatc cgggacttgc tatacattgg caaggatcgc 1200

aaaaacccaa gggaggatta tctggatgtc tatgtgtttg gggtcggccc tttggtgaac 1260

caagtgaaca tcaatgcttt ggcttccaag aaagacaatg agcaacatgt gttcaaagtc 1320

aaggatatgg aaaacctgga agatgttttc taccaaatga tcgatgaaag ccagtctctg 1380

agtctctgtg gcatggtttg ggaacacagg aagggtaccg attaccacaa gcaaccatgg 1440

caggccaaga tctcagtcat tcgcccttca aagggacacg agagctgtat gggggctgtg 1500

gtgtctgagt actttgtgct gacagcagca cgttgtttca ctgtggatga caaggaacac 1560

tcaatcaagg tcagcgtagg aggggagaag cgggacctgg agatagaagt agtcctattt 1620

caccccaact acaacattaa tgggaaaaaa gaagcaggaa ttcctgaatt ttatgactat 1680

gacgttgccc tgatcaagct caagaataag ctgaaatatg gccagactat caggcccatt 1740

tgtctcccct gcaccgaggg aacaactcga gctttgaggc ttcctccaac taccacttgc 1800

cagcaacaaa aggaagagct gctccctgca caggatatca aagctctgtt tgtgtctgag 1860

gaggagaaaa agctgactcg gaaggaggtc tacatcaaga atggggataa gaaaggcagc 1920

tgtgagagag atgctcaata tgccccaggc tatgacaaag tcaaggacat ctcagaggtg 1980

gtcacccctc ggttcctttg tactggagga gtgagtccct atgctgaccc caatacttgc 2040

agaggtgatt ctggcggccc cttgatagtt cacaagagaa gtcgtttcat tcaagttggt 2100

gtaatcagct ggggagtagt ggatgtctgc aaaaaccaga agcggcaaaa gcaggtacct 2160

gctcacgccc gagactttca catcaacctc tttcaagtgc tgccctggct gaaggagaaa 2220

ctccaagatg a 2231

<210> 61
<211> 2361
<212> DNA
<213> Artificial Sequence

<220>
<223> FLAG - hFurin - His8

<400> 61
gactataaag atgacgacga caagcaaggt caaaaggtat ttaccaatac atgggcagtc 60

agaattccgg gaggaccagc agtggccaat tcggttgcta ggaagcatgg ttttctgaac 120

ttgggtcaaa tcttcggtga ctactatcat ttctggcaca gaggtgtcac aaaacgatct 180

ttaagtcccc ataggccaag acattcaagg ctacagagag aaccacaggt ccagtggctg 240

gaacagcaag ttgctaagcg tagaacaaaa agagatgtat accaggaacc taccgatcca 300

aagtttcccc agcaatggta cctctcaggt gtaactcaaa gagatcttaa tgttaaagca 360

gcctgggctc agggatatac tggtcatggt atagtggttt ccatcctaga tgatggtata 420

gagaaaaatc atcctgacct ggccggtaat tacgacccag gtgctagttt cgatgttaac 480

gatcaggacc cagatccaca acctaggtat acccaaatga atgataacag acatggaaca 540

cgttgtgctg gagaggtcgc tgctgttgca aataacggtg tctgtggggt tggcgttgct 600

tacaacgctc gtattggtgg agtgagaatg ttggacggtg aagtgactga tgctgtggaa 660

gctagatctt tgggattgaa tcctaatcat atccacattt actcagcaag ttggggtcca 720

gaagatgacg gcaaaacagt tgacggacca gcacgtcttg ccgaggaggc attctttcgt 780

ggtgtaagtc aaggaagggg tggtttgggc tctattttcg tttgggcctc cggtaacggt 840

ggtagagaac atgattcctg caactgtgac ggttatacca acagcatcta taccttatct 900

atttcatccg caactcaatt tggaaatgtg ccatggtatt cagaagcttg ttcttctact 960

ctggcgacaa catactcctc gggtaatcaa aatgagaaac aaatcgtcac taccgacctt 1020

cgtcaaaagt gcactgaatc tcacacaggt acatctgctt ccgctcctct ggcagccggt 1080

atcatcgctt taactttaga agcaaacaaa aacctgactt ggagagacat gcaacacctc 1140

gtggtccaga catcgaagcc agctcactta aatgctaacg attgggctac taatggagta 1200

ggaaggaaag tttcgcattc atacggttat ggtcttttgg atgcaggagc aatggtggca 1260

ttggcacaaa attggactac tgtcgcacca cagagaaaat gtattatcga tatcctaact 1320

gagccaaagg acattggtaa acgtttggaa gtgagaaaaa cagttactgc ttgtctgggc 1380

gaaccaaatc atataactcg tttggaacac gctcaagcaa gattgactct atcttacaat 1440

agaagagggg atctagctat tcatttagtt tctcccatgg gaactagatc aacactgttg 1500

gctgccagac ctcatgacta ttccgctgat ggtttcaatg actgggcctt tatgactacc 1560

cacagctggg acgaagatcc ttccggtgag tgggttctgg aaattgagaa cacatccgaa 1620

gctaacaatt acggtacgct tacgaagttc acattggttt tgtacggaac ggctcctgaa 1680

ggcctgccgg ttcctcctga gagtagcggt tgcaagactt tgacttccag tcaagcatgt 1740

gttgtgtgtg aggagggttt ctcccttcat caaaagtctt gtgtgcagca ttgtccacca 1800

ggatttgccc cacaggtgtt ggatacccac tattcaacag agaatgacgt tgagacaatc 1860

agagcctctg tatgtgctcc ctgtcacgca tcctgtgcta cttgtcaagg acccgctctt 1920

acggattgct tgtcctgtcc atcccacgca agcttggacc cggtagagca aacttgttcc 1980

agacaatcac aatccagtcg tgaatcccca ccacaacaac aacctccaag attgcctcct 2040

gaggtggaag ctggtcaaag acttagagct ggattgctcc cttctcattt gcctgaggtc 2100

gtagctggtt tgagctgcgc cttcattgtg ctggtttttg tcactgtttt cttagttttg 2160

cagttacgta gcggattttc atttcgagga gtcaaagttt acacgatgga caggggtttg 2220

atctcataca agggtctgcc ccctgaggca tggcaggagg agtgcccttc ggacagcgag 2280

gaagatgaag gcagaggtga acgtacagca ttcatcaagg atcaatcagc tttgcaccac 2340

caccatcatc accatcacta a 2361

<210> 62
<211> 6075
<212> DNA
<213> Artificial Sequence

<220>
<223> Cas9GFP-gRNA

<400> 62
agatcttttt tgtagaaatg tcttggtgtc ctcgtccaat caggtagcca tctctgaaat 60

atctggctcc gttgcaactc cgaacgacct gctggcaacg taaaattctc cggggtaaaa 120

cttaaatgtg gagtaatgga accagaaacg tctcttccct tctctctcct tccaccgccc 180

gttaccgtcc ctaggaaatt ttactctgct ggagagcttc ttctacggcc cccttgcagc 240

aatgctcttc ccagcattac gttgcgggta aaacggaggt cgtgtacccg acctagcagc 300

ccagggatgg aaaagtcccg gccgtcgctg gcaataatag cgggcggacg catgtcatga 360

gattattgga aaccaccaga atcgaatata aaaggcgaac acctttccca attttggttt 420

ctcctgaccc aaagacttta aatttaattt atttgtccct atttcaatca attgaacaac 480

tatttcgaaa acgatggaca agaagtactc cattgggctc gatatcggca caaacagcgt 540

cggctgggcc gtcattacgg acgagtacaa ggtgccgagc aaaaaattca aagttctggg 600

caataccgat cgccacagca taaagaagaa cctcattggc gccctcctgt tcgactccgg 660

ggagacggcc gaagccacgc ggctcaaaag aacagcacgg cgcagatata cccgcagaaa 720

gaatcggatc tgctacctgc aggagatctt tagtaatgag atggctaagg tggatgactc 780

tttcttccat aggctggagg agtccttttt ggtggaggag gataaaaagc acgagcgcca 840

cccaatcttt ggcaatatcg tggacgaggt ggcgtaccat gaaaagtacc caaccatata 900

tcatctgagg aagaagcttg tagacagtac tgataaggct gacttgcggt tgatctatct 960

cgcgctggcg catatgatca aatttcgggg acacttcctc atcgaggggg acctgaaccc 1020

agacaacagc gatgtcgaca aactctttat ccaactggtt cagacttaca atcagctttt 1080

cgaagagaac ccgatcaacg catccggagt tgacgccaaa gcaatcctga gcgctaggct 1140

gtccaaatcc cggcggctcg aaaacctcat cgcacagctc cctggggaga agaagaacgg 1200

cctgtttggt aatcttatcg ccctgtcact cgggctgacc cccaacttta aatctaactt 1260

cgacctggcc gaagatgcca agcttcaact gagcaaagac acctacgatg atgatctcga 1320

caatctgctg gcccagatcg gcgaccagta cgcagacctt tttttggcgg caaagaacct 1380

gtcagacgcc attctgctga gtgatattct gcgagtgaac acggagatca ccaaagctcc 1440

gctgagcgct agtatgatca agcgctatga tgagcaccac caagacttga ctttgctgaa 1500

ggcccttgtc agacagcaac tgcctgagaa gtacaaggaa attttcttcg atcagtctaa 1560

aaatggctac gccggataca ttgacggcgg agcaagccag gaggaatttt acaaatttat 1620

taagcccatc ttggaaaaaa tggacggcac cgaggagctg ctggtaaagc ttaacagaga 1680

agatctgttg cgcaaacagc gcactttcga caatggaagc atcccccacc agattcacct 1740

gggcgaactg cacgctatcc tcaggcggca agaggatttc tacccctttt tgaaagataa 1800

cagggaaaag attgagaaaa tcctcacatt tcggataccc tactatgtag gccccctcgc 1860

ccggggaaat tccagattcg cgtggatgac tcgcaaatca gaagagacca tcactccctg 1920

gaacttcgag gaagtcgtgg ataagggggc ctctgcccag tccttcatcg aaaggatgac 1980

taactttgat aaaaatctgc ctaacgaaaa ggtgcttcct aaacactctc tgctgtacga 2040

gtacttcaca gtttataacg agctcaccaa ggtcaaatac gtcacagaag ggatgagaaa 2100

gccagcattc ctgtctggag agcagaagaa agctatcgtg gacctcctct tcaagacgaa 2160

ccggaaagtt accgtgaaac agctcaaaga agactatttc aaaaagattg aatgtttcga 2220

ctctgttgaa atcagcggag tggaggatcg cttcaacgca tccctgggaa cgtatcacga 2280

tctcctgaaa atcattaaag acaaggactt cctggacaat gaggagaacg aggacattct 2340

tgaggacatt gtcctcaccc ttacgttgtt tgaagatagg gagatgattg aagaacgctt 2400

gaaaacttac gctcatctct tcgacgacaa agtcatgaaa cagctcaaga ggcgccgata 2460

tacaggatgg gggcggctgt caagaaaact gatcaatggg atccgagaca agcagagtgg 2520

aaagacaatc ctggattttc ttaagtccga tggatttgcc aaccggaact tcatgcagtt 2580

gatccatgat gactctctca cctttaagga ggacatccag aaagcacaag tttctggcca 2640

gggggacagt cttcacgagc acatcgctaa tcttgcaggt agcccagcta tcaaaaaggg 2700

aatactgcag accgttaagg tcgtggatga actcgtcaaa gtaatgggaa ggcataagcc 2760

cgagaatatc gttatcgaga tggcccgaga gaaccaaact acccagaagg gacagaagaa 2820

cagtagggaa aggatgaaga ggattgaaga gggtataaaa gaactggggt cccaaatcct 2880

taaggaacac ccagttgaaa acacccagct tcagaatgag aagctctacc tgtactacct 2940

gcagaacggc agggacatgt acgtggatca ggaactggac atcaatcggc tctccgacta 3000

cgacgtggat catatcgtgc cccagtcttt tctcaaagat gattctattg ataataaagt 3060

gttgacaaga tccgataaaa atagagggaa gagtgataac gtcccctcag aagaagttgt 3120

caagaaaatg aaaaattatt ggcggcagct gctgaacgcc aaactgatca cacaacggaa 3180

gttcgataat ctgactaagg ctgaacgagg tggcctgtct gagttggata aagccggctt 3240

catcaaaagg cagcttgttg agacacgcca gatcaccaag cacgtggccc aaattctcga 3300

ttcacgcatg aacaccaagt acgatgaaaa tgacaaactg attcgagagg tgaaagttat 3360

tactctgaag tctaagctgg tctcagattt cagaaaggac tttcagtttt ataaggtgag 3420

agagatcaac aattaccacc atgcgcatga tgcctacctg aatgcagtgg taggcactgc 3480

acttatcaaa aaatatccca agcttgaatc tgaatttgtt tacggagact ataaagtgta 3540

cgatgttagg aaaatgatcg caaagtctga gcaggaaata ggcaaggcca ccgctaagta 3600

cttcttttac agcaatatta tgaatttttt caagaccgag attacactgg ccaatggaga 3660

gattcggaag cgaccactta tcgaaacaaa cggagaaaca ggagaaatcg tgtgggacaa 3720

gggtagggat ttcgcgacag tccggaaggt cctgtccatg ccgcaggtga acatcgttaa 3780

aaagaccgaa gtacagaccg gaggcttctc caaggaaagt atcctcccga aaaggaacag 3840

cgacaagctg atcgcacgca aaaaagattg ggaccccaag aaatacggcg gattcgattc 3900

tcctacagtc gcttacagtg tactggttgt ggccaaagtg gagaaaggga agtctaaaaa 3960

actcaaaagc gtcaaggaac tgctgggcat cacaatcatg gagcgatcaa gcttcgaaaa 4020

aaaccccatc gactttctcg aggcgaaagg atataaagag gtcaaaaaag acctcatcat 4080

taagcttccc aagtactctc tctttgagct tgaaaacggc cggaaacgaa tgctcgctag 4140

tgcgggcgag ctgcagaaag gtaacgagct ggcactgccc tctaaatacg ttaatttctt 4200

gtatctggcc agccactatg aaaagctcaa agggtctccc gaagataatg agcagaagca 4260

gctgttcgtg gaacaacaca aacactacct tgatgagatc atcgagcaaa taagcgaatt 4320

ctccaaaaga gtgatcctcg ccgacgctaa cctcgataag gtgctttctg cttacaataa 4380

gcacagggat aagcccatca gggagcaggc agaaaacatt atccacttgt ttactctgac 4440

caacttgggc gcgcctgcag ccttcaagta cttcgacacc accatagaca gaaagcggta 4500

cacctctaca aaggaggtcc tggacgccac actgattcat cagtcaatta cggggctcta 4560

tgaaacaaga atcgacctct ctcagctcgg tggagacagc agggctgacc ccaagaagaa 4620

gaggaaggtg tctaaaggtg aagaattatt cactggtgtt gtcccaattt tggttgaatt 4680

agatggtgat gttaatggtc acaaattttc tgtctccggt gaaggtgaag gtgatgctac 4740

ttacggtaaa ttgaccttaa aatttatttg tactactggt aaattgccag ttccatggcc 4800

aaccttagtc actactttcg gttatggtgt tcaatgtttt gctagatacc cagatcatat 4860

gaaacaacat gactttttca agtctgccat gccagaaggt tatgttcaag aaagaactat 4920

ttttttcaaa gatgacggta actacaagac cagagctgaa gtcaagtttg aaggtgatac 4980

cttagttaat agaatcgaat taaaaggtat tgattttaaa gaagatggta acattttagg 5040

tcacaaattg gaatacaact ataactctca caatgtttac atcatggctg acaaacaaaa 5100

gaatggtatc aaagttaact tcaaaattag acacaacatt gaagatggtt ctgttcaatt 5160

agctgaccat tatcaacaaa atactccaat tggtgatggt ccagtcttgt taccagacaa 5220

ccattactta tccactcaat ctgccttatc caaagatcca aacgaaaaga gagaccacat 5280

ggtcttgtta gaatttgtta ctgctgctgg tattacccat ggtatggatg aattgtacaa 5340

atgagtttta gccttagaca tgactgttcc tcagttcaag ttgggcactt acgagaagac 5400

cggtcttgct agattctaat caagaggatg tcagaatgcc atttgcctga gagatgcagg 5460

cttcattttt gatacttttt tatttgtaac ctatatagta taggattttt tttgtcattt 5520

tgtttcttct cgtacgagct tgctcctgat cagcctatct cgcagctgat gaatatcttg 5580

tggtaggggt ttgggaaaat cattcgagtt tgatgttttt cttggtattt cccactcctc 5640

ttcagagtac agaagattaa gtgagacctt cgtttgtgcg gatcctcttt gaaaagataa 5700

tgtatgatta tgctttcact catatttata cagaaacttg atgttttctt tcgagtatat 5760

acaaggtgat tacatgtacg tttgaagtac aactctagat tttgtagtgc cctcttgggc 5820

tagcggtaaa ggtgcgcatt ttttcacacc ctacaatgtt ctgttcaaaa gattttggtc 5880

aaacgctgta gaagtgaaag ttggtgcgca tgtttcggcg ttcgaaactt ctccgcagtg 5940

aaagataaat gatccaacaa tcattcgacc tagctggttt tagagctaga aatagcaagt 6000

taaaataagg ctagtccgtt atcaacttga aaaagtggca ccgagtcggt ggtgcttttt 6060

ttgtttttta tgtct 6075